# EUROPEAN PATENT APPLICATION

(11) **EP 2 210 896 A1**
(43) Date of publication of application: **28.07.2010**
(21) Application number: 08834813.1
(22) Date of filing: 03.10.2008
(51) Int. Cl.: C07H 5/02, C07H 13/06, C07H 15/04, C07H 19/067

(54) **PROCESS FOR PRODUCTION OF RIBOFURANOSE DERIVATIVES**

(30) Priority: 05.10.2007 JP 2007261962; 06.06.2008 JP 2008149093
(71) Applicant: API Corporation, Osaka-shi, Osaka 541-0046 (JP)
(72) Inventor: MAEDA, Tomoko, Yokohama-shi Kanagawa 227-8502 (JP); UEHARA, Hisatoshi, Yokohama-shi Kanagawa 227-8502 (JP); HARADA, Naoki, Yokohama-shi Kanagawa 227-8502 (JP); KATSURADA, Manabu, Yokohama-shi Kanagawa 227-8502 (JP); SANO, Mitsuharu, Kitakyushu-shi Fukuoka 806-0004 (JP)
(74) Representative: Polypatent
(86) International application number: PCT/JP2008/068095
(87) International publication number: WO 2009/044886

(57) **Abstract**

It is an object of the present Invention to provide a process for producing 1,2,3-tri-O-acetyl-5-deoxy-ribofuranose in an industrially appropriate manner. The present invention provides a process for producing a 1,2,3-tri-O-acetyl-5-deoxy-ribofuranose which comprises hydrogenating a compound represented by the formula (1) or formula (2) in the presence of a metal catalyst: 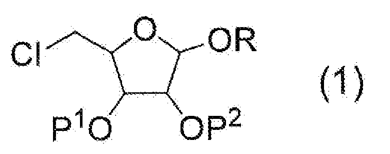 wherein P¹ and P² independently represent a hydrogen atom or an acyl group OP¹ and OP² may together form an acetal group, and R represents a hydrogen atom, an alkyl group, an aryl group, an aralkyl group, or an acyl group; 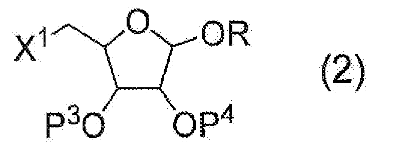 wherein X¹ represents Br or I, P³ and P⁴ independently represent a hydrogen atom or an acryl group, and R represents a hydrogen atom, an alkyl group, an aryl group, an aralkyl group, or an acyl group.

## Description

### Technical Field

The present invention relates to a process for producing ribofuranose derivatives. The furanose derivatives produced by the process of the present invention are useful as synthetic intermediates of nucleic acid derivatives that are pharmacologically active substances.

### Background Art

Hitherto, only one example of a process for producing 1,2,3-tri-O-acetyl-5-deoxyribofuranose has been reported, which is a process that involves the use of, as an intermediate, a compound containing a ribose having hydroxyl groups protected with cyclic acetal at 2- and 3-positions(see Non-Patent Documents I to 5 and Patent Documents 1 to 3). However, in order to produce an intermediate of medicine, protection and deprotection of hydroxyl groups require the use of reagents. In addition, the process comprises complicated operations and time-consuming production steps. Therefore, such process is not preferable for the purpose of achieving inexpensive and simple production process.

In each of the above documents, in order to protect hydroxyl groups at 2- and 3-positions of ribose with cyclic acetal, a reagent or solvent (e.g., 2, 2-dimethoxypropane and acetone) is used in a large amount, a hydroxyl group at 5-position is converted into a functional group, and then 2,3-cyclic acetal is deprotected. Thus, 5-deoxy-ribofuranose is obtained. For deprotection of 2,3-cyclic acetal, a large amount of water is necessary. However, 5-deoxy-ribofuranose is a highly polar substance and therefore cannot be obtained via extraction. Therefore, it is necessary to remove water by distillation from an aqueous solution in order to obtain 5-deoxy-ribofuranose. Removal of a large amount of water by distillation cannot be efficiently carried out at industrial scales. In addition, it is difficult to achieve complete removal of water. In such case, for example, it is necessary to carry out azeotropic dehydration with the use of a large amount of a solvent and to use an excessive amount of a reagent in the subsequent acetylation step, which is problematic.

In addition to the above problems, known production processes with the use of a 2,3-cyclic acetal compound as an intermediate have the following problems. In a production process that involves the use of a 5-O-tosyl compound as an intermediate (see Non-Patent Documents 1 and 2), methylene chloride, which is an environmentally problematic substance, is used as a solvent for tosylation of a hydroxyl group at 5-position. Also, pyridine, which is more expensive than general solvents, needs to be subjected to complicated aftertreatment, and is problematic when discarded, is used as a reagent and solvent in a large amount, which is not industrially preferable. Further, upon reduction of a tosyloxy group at 5-position, hydrogenated metal reagents such as sodium borohydride and lithium aluminium hydride are used in large amounts. However, these reagents are water-reactive reagents that spontaneously combust and thus have high risks of explosion. Therefore, handling, use and after-treatment of such reagents are difficult in industrial-scale.

In addition, there are disclosed processes for induction of a pharmaceutical compound with the use of, as an intermediate, a 5-deoxyribofuranose derivative in which hydroxyl groups at 2- and 3-positions are protected with cyclic acetal and a benzoyl group (see Non-Patent Document 6, Patent Document 4, and Patent Document 5).

In a process that involves the use of a 5-bromo compound as an intermediate (see Non-Patent Document 3 and Patent Documents 1 and 2), triphenylphosphine is used in an excessive amount for bromination of a hydroxyl group at 5-position and thus a large amount of triphenylphosphine oxide is obtained as a by-product after reaction. For industrial production, generation of a product that is not intended to be produced, that is to say, a by-product, is problematic in view of costs and the environment. In addition, purification with silica gel is necessary for isolation of a desired 5-bromo compound with high purity from a reaction solution, Therefore, the above process is not industrially applicable. Further, in order to cause a reaction to proceed smoothly, it is necessary to use methylene chloride which causes environmental problems in a large amount, which is not industrially preferable.

In a process that involves the use of a 5-iodine compound as an intermediate (see Non-Patent Documents 4 and 5 and Patent Document 4), it is necessary to conduct a two-stage step in which a hydroxyl group at 5-position is first subjected to tosylation or mesylation, followed by iodination with sodium iodide. Therefore, the process has problems similar to those in the case of the process that involves the use of a 5-O-tosyl compound as an intermediate. Since it is necessary to carry out a multi-stage operation, further expensive sodium iodide needs to be used in an excessively large amount. Therefore, the production process is not appropriate for industrial production.

In a process that involves the use of a 5-chloro compound as an intermediate (see Patent Document 3), triphenylphosphine is used. Therefore, the process has problems similar to those in the case of the process that involves the use of a 5-bromo compound upon chlorination of a hydroxyl group at 5-position. In addition, for reduction of a chloro group, radical reduction is conducted with the use of trialkyl-tin hydride, which is toxic and environmentally problematic. Therefore, it is difficult to conduct such process for industrial purposes.

Meanwhile, an example of a process wherein a hydroxyl group at 5-position of ribose is directly subjected to chlorination without protection of hydroxyl groups at 2- and 3-positions of ribose with cyclic acetal has been reported (see Non-Patent Document 6 and Patent Document 5). However, synthesis of 5-chloro compound requires a dehydration step involving the use of a desiccant, purification with silica gel, and the like. Also, for aftertreatment, it is necessary to carry out an operation for concentrating a large amount of water. In addition, a large amount of an extraction solvent is used to obtain a highly water-soluble product of interest. The process is not an inexpensive and simple industrial production process.

In addition, for industrial production, a solid product is effective in terms of better handleability than in the case of a liquid-solid mixture in view of ease of quality control. In a process for producing 1,2,3-tri-O-acetyl-5-deoxyribofuranose that has been reported, a final product is a mixture of α-anomer in a liquid form and β-anomer in a solid form and therefore a crystallization operation or the like is necessary for obtaining β-anomer. Upon such crystallization operation, α-anomer is removed, resulting in a decrease in the total yield, which is problematic.

Further, in recent years, a process for producing 1,2,3-tri-O-acetyl-5-deoxyribofuranose with the use of 5-deoxyribofuranose as an intermediate and natural inosine as a starting material has become known (Patent Document 6). In this process, imidazoylinosine, triphenylphosphine, and iodine are used in amounts equivalent to or greater than the amount of inosine used as a starting material for iodination of a hydroxyl group at 5-position of inosine, resulting in high cost of production of 1,2,3-tri-O-acetyl-5-deoxyribofuranose. In addition, triphenylphosphine oxide is generated in a large amount as a by-product after reaction, which is problematic. Further, the reaction time of reduction of 5-iodine deoxyinosine takes as long as 12 to 24 hours. Therefore, the above process is not an appropriate industrial production process.

In addition to the above, as a process for producing a furanose derivative having a deoxylated group at 5-position, a process wherein 5-bromo-xylofuranose is hydrogenated in the presence of a palladium catalyst has been reported. However, the report merely discloses an example of reduction of 5-bromo-xylofuranose, but no examples of reduction of 5-chloro-xylofuranose (Non-Patent Document 7). In general, the degree of difficulty of reduction of an organic halogen compound via hydrogenation (hydrogenolysis, dehalogenation reaction, etc.) would vary depending on halogen element type and reaction substrate structure. Regarding carbon-halogen bonds, reduction (hydrogenolysis, dehalogenation reaction, etc.) via hydrogenation becomes difficult in the order of carbon-iodine > carbon-bromine > carbon-chlorine > carbon-fluorine. The intensities of dissociation energy of carbon-halogen bonds are in the following order: carbon-iodine (222,6 kJ/mol), carbon-bromine (281.4 kJ/mol), carbon-chlorine (340.2 kJ/mol), and carbon-fluorine (453.6 kJ/mol). This is the reverse order of susceptibility to reduction via hydrogenation. Therefore, it is understood that it is difficult to reduce a carbon-chlorine bond via hydrogenation when the dissociation energy of the bond is larger than that of a carbon-iodine or carbon-bromine bond. Particularly in the case of a substrate of 5-chloro-xylofuranose, 5-chloro-ribofuranose, or the like, a carbon atom at α-position, which is bound to a chloride atom, forms an electron-donating ether bond with an adjacent oxygen atom. Therefore, the dissociation energy of a carbon-chlorine bond further increases. In addition, secondary carbon at α-position is sterically bulky so that a metal catalyst used for hydrogenation is unlikely to be inserted into a carbon-chlorine bond. Accordingly, reduction becomes less likely to proceed.
Non-Patent Document 1: P. Sairam et al., Carbohydrate Research, 2003, vol. 338, no. 4, pp. 303-306
Non-Patent Document 2: G. Wang et al., Journal of Medicinal Chemistry, 2000, vol. 43, no. 13, pp. 2566-2574
Non-Patent Document 3: K. S. Ramasamy et al., Journal of Medicinal Chemistry, 2000, vol. 43, no. 5, pp. 1019-1028
Non-Patent Document 4: H. M. Kissman et al., Journal of American Chemical Society, 1957, vol. 79, no. 20, pp. 5534-5540
Non-Patent Document 5: Q-H. Zheng et al., Nuclear Medicine and Biology, 2004, vol. 31, no. 8, pp. 1033-1041
Non-Patent Document 6: H. B. Cottam et al., Journal of Medicinal Chemistry, 1993, vol. 36, no. 22, pp. 3424-3430
Non-Patent Document 7: H. David et al., Carbohydrate Research, 1975, vol. 42, no. 2, pp. 241-249
Patent Document 1: EP Patent No. 21231
Patent Document 2: JP Patent Publication (Kokai) No. 56-005497 A (1981)
Patent Document 3: WO97/25337
Patent Document 4: US Patent No. 2,847,413
Patent Document 5: WO94/06438
Patent Document 6: CN Patent Application No. CN 101012252A

### Disclosure of the Invention

### Problem to be Solved by the Invention

It is an object of the present invention to provide a process for producing 1,2,3-tri-O-acetyl-5-deoxy-ribofuranose in an industrially appropriate manner so as to obtain β-anomer thereof at a high yield.

### Means for Solving Problem

As a result of intensive studies in order to achieve the above object, the present inventors have completed a process for producing 1,2,3-tri-O-acetyl-5-deoxyribofuranose which comprises reducing a 5-halogeno-5-deoxyribofuranose derivative obtained without a complicated form of purification, via hydrogenation.

Thus, the present invention provides the followings.
(1) A process for producing a compound represented by the formula (3): 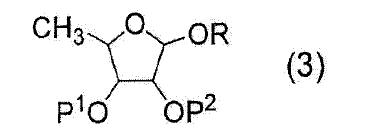 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
   which comprises hydrogenating a compound represented by the formula (1) or the formula (2) in the presence of a metal catalyst: 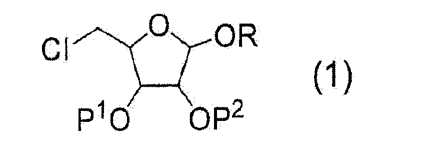 wherein P¹ and P² independently represent a hydrogen atom or an acyl group, OP¹ and OP² may together form an acetal group, and R represents a hydrogen atom, an alkyl group, an aryl group, an aralkyl group, or an acyl group; 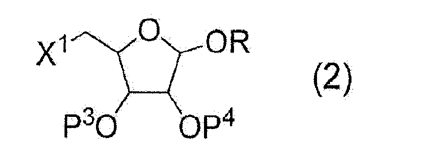 wherein X¹ represents Br or I, P³ and P⁴ independently represent a hydrogen atom or an acyl group, and R represents a hydrogen atom, an alkyl group, an aryl group, an aralkyl group, or an acyl group.
(2) The process according to (1), wherein P¹ and P² independently represent a hydrogen atom or an acyl group and P³ and P⁴ independently represent a hydrogen atom or an acyl group in the formula (1) or (2).
(3) The process according to (1) or (2), wherein a hydrogen molecule is allowed to act in the presence of the metal catalyst for hydrogenation.
(4) A process for producing a compound represented by the formula (3): 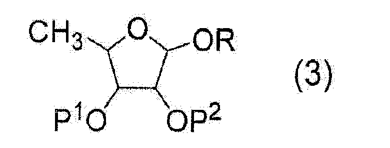 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
   which comprises the following steps of:
   (a) reacting a compound represented by the formula (4): 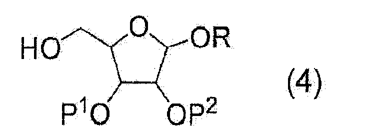 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
      with an acid halide or a halogen salt of an acid halide and an alkali metal, and treating the resultant with an acid or an alkali so as to produce a compound represented by the formula (5); 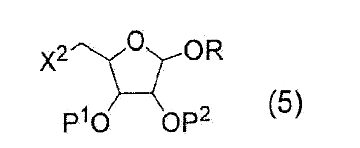 wherein X² represents Cl, Br, or I, P¹ and P² independently represent a hydrogen atom or an acyl group, OP¹ and OP² may together form an acetal group, and R represents a hydrogen atom, an alkyl group, an aryl group, an aralkyl group, or an acyl group; and
   (b) hydrogenating the compound represented by the formula (5): 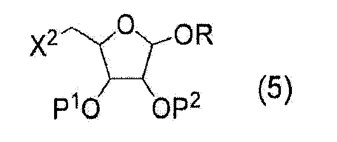 wherein X² represents Cl, Br, or I, P¹ and P² independently represent a hydrogen atom or an acyl group, OP¹ and OP² may together form an acctal group, and R represents a hydrogen atom, an alkyl group, an aryl group, an aralkyl group, or an acyl group; in the presence of a metal catalyst.
(5) A process for producing a compound represented by the formulat (6); 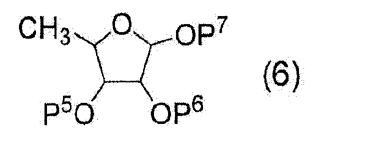 wherein P⁵, P⁶, and P⁷ independently represent an acyl group and may be the same or different;
   which comprises the following steps of:
   (a) producing a compound represented by the formula (3); 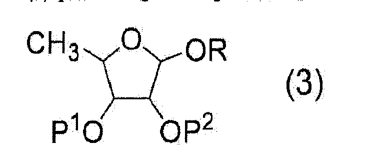 wherein P¹ and P² independently represent a hydrogen atom or an acyl group, OP¹ and OP² may together form an acetal group, and R represents a hydrogen atom, an alkyl group, an aryl group, an aralkyl group, or an acyl group, provided that P¹, P², and R do not simultaneously represent an acyl group;
      by the process according to any one of (1) to (4); and
   (b) converting a hydroxyl group or substituted hydroxyl group in the compound represented by the formula (3) into a hydroxyl groupe substituted with an acyl group.
(6) A process for producing a compound represented by the formula (8); 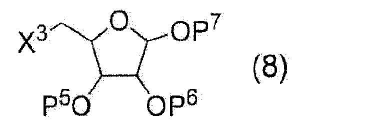 wherein X³ represents Cl, Br, or I and P⁵, P⁶, and P⁷ independently represent an acyl group and may be the same or different;
   which comprises the following steps of:
   (a) reacting a compound represented by the formula (4): 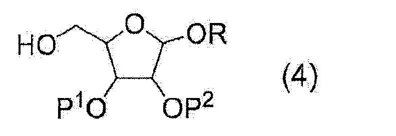 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
      with an acid halide or a halogen salt of an acid halide of an alkali metal, and treating the resultant with an acid or an alkali so as to produce a compound represented by the formula (7); 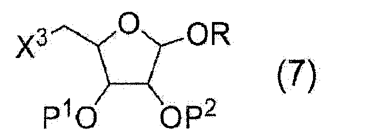 wherein X³ represents Cl, Br, or I, P¹ and P² independently represent a hydrogen atom or an acyl group, OP¹ and OP² may together form an acetal group, and R represents a hydrogen atom, an alkyl group, an aryl group, an aralkyl group, or an acyl group, provided that P¹, P², and R do not simultaneously represent an acyl group; and
   (b) converting a hydroxyl group or substituted hydroxyl group of the compound represented by the formula (7) into a hydroxyl group substituted with an acyl group.
(7) A process for producing a nucleic acid derivative of the formula (9): 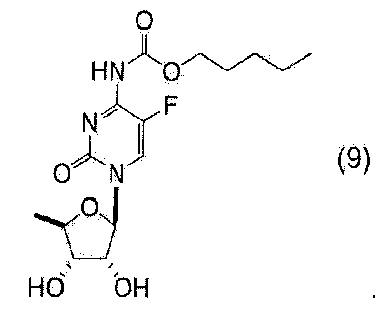 which comprises the following steps of:
   (a) producing a compound represented by the formula (6):

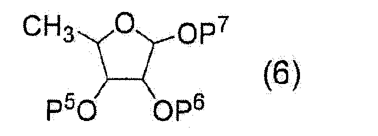

      wherein P⁵, P⁶, and P⁷ independently represent an acyl group and may be the same or different;
      by the process according to (5); and
   (b) condensing the compound represented by said formula (6) obtained in the step (a) with 5-fluorocylosines.
(8) A process for producing a mixture containing an α-anomer and a β-anomer of a compound of the formula (10); 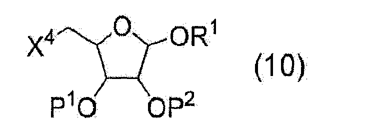 wherein X⁴ represents Cl, Br, I, or a hydrogen atom, P¹ and P² independently represent a hydrogen atom or an acyl group, OP¹ and OP² may together form an acetal group, and R¹ represents an acyl group,
   which comprises treating a mixture containing a compound of said formula (10) with an α-configuration at 1-position (α-anomer) and a compound of said formula (10) with a β-configuration at 1-position (β-anomer) in the presence of an acid and a poor solvent, wherein the proportion of the β-anomer in the mixture after treatment becomes greater than that in the mixture before treatment.
(9) The process according to (8), wherein a base is further allowed to exist during the treatment in the presence of the acid and the poor solvent.
(10) The process according to (8) or (9), wherein a dehydration agent is further allowed to exist during the treatment in the presence of the acid and the poor solvent.
(11) A process for producing a β-anomer of a compound of the formula (10): 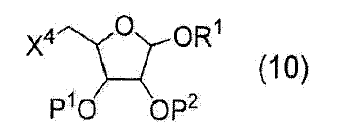 wherein X⁴ represents Cl, Br, I, or a hydrogen atom, P¹ and P² independently represent a hydrogen atom or an acyl group, OP¹ and OP² may together form an acetal group, and R¹ represents an acyl group,
   which comprising the following steps of:
   (a) producing a mixture containing the compound of said formula (10) with an α-configuration at 1-position (α-anomer) and the compound of said formula (10) with a β-configuration at 1-position (β-anomer) by the process according to any one of (8) to (10);and
   (b) isolating the β-anomer of a compound of said formula (10) by further purifying the mixture containing an α-anomer and a β-anomer of a compound of said formula (10) produced in the step (a).
(12) A process for producing a compound of the formula (10); 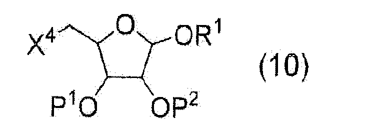 wherein X⁴ represents Cl, Br, I, or a hydrogen atom, P¹ and P² independently represent a hydrogen atom or an acyl group, OP¹ and OP² may together form an acetal group, and R¹ represents an acyl group;
   which comprises a step of allowing an acylation agent to act on a mixture containing a compound of the formula (11): 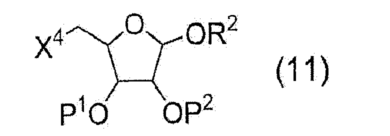 wherein X⁴ represents Cl, Br, I, or a hydrogen atom, P¹ and P² independently represent a hydrogen atom or an acyl group, OP¹ and OP² may together form an acetal group, and R² represents an alkyl group, an aryl group, or an aralkyl group;
   with an α-configuration at 1-position (α-anomer) and a compound of said formula (11) with a β-configuration at 1-position (β-anomer) in the presence of an acid and a poor solvent before reaction, so as to obtain a mixture containing the compound of said formula (10) with an α-configuration at 1-position (α-anomer) and the compound of said formula (10) with a β-configuration at 1-position (β-anomer) after reaction, wherein the proportion of the β-anomer in the mixture after reaction becomes greater than that in the mixture before reaction:
(13) The process according to (12), wherein a base is further allowed to exist when the acylation agent is allowed to act in the presence of the acid and the poor solvent.
(14) A process for producing a β-anomer of a compound of the formula (10): 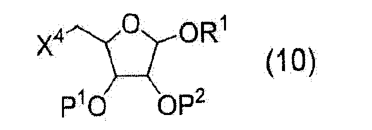 wherein X⁴ represents Cl, Br, I, or a hydrogen atom, P¹ and P² independently represent a hydrogen atom or an acyl group, OP¹ and OP² may together form an acetal group, and R¹ represents an acyl group;
   which comprises the following steps of:
   (a) producing a mixture containing the compound of said formula (10) with an α-configuration at 1-position (α-anomer) and the compound of said formula (10) with a β-configuration at 1-position (β-anomer) by the process of (12) or (13); and
   (b) isolating the β-anomer of a compound of said formula (10) by further purifying the mixture containing an α-anomer and a β-anomer of a compound of said formula (10) produced in the step (a).
(15) A D- or L-ribofuranose derivative represented by the formula (12), 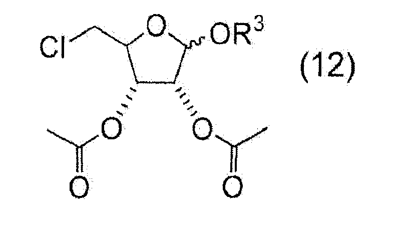
wherein a configuration of 1-position is α or β and R³ represents an alkyl group with a carbon number of 1 to 6, an aryl group with a carbon number of 6 to 20, or an aralkyl group with a carbon number of 7 to 12.

### Effects of the Invention

According to the present invention, 1,2,3-tri-O-acetyl-5-deoxyribofuranose, which is a ribose derivative useful as an intermediate of medicine, can be obtained by an industrially appropriate process. According to the present invention, 5-deoxy-ribofuranose, 5-halogeno-5-deoxy-ribofuranose; acylated 5-deoxy-ribofuranose, or 5-halogeno-5-deoxy-ribofuranose can be obtained by an industrially appropriate process with good efficiency. In addition, according to the present invention, a novel 1-O-methyl-2,3-di-O-acetyl-5-deoxy-5-chloro-D-ribofuranose, which can be induced to become 1,2,3-tri-O-acetyl-5-deaxyribofuranose, can be obtained with good efficiency. Further, according to the present invention, β-anomer of 1,2,3-tri-O-acetyl-5-deoxy-ribofuranose can be obtained at a high yield. 1,2,3-tri-O-acetyl-5-deoxyribofuranose that can be obtained by the process of the present invention can be induced to become Capecitabine, which is a nucleic acid derivative used as a medicine known to be useful as an anticancer agent, described in, for example, Bioorganic & Medicinal Chemistry, 2000, vol. 8, no. 8, pp. 1967-1706.

### Best Mode for Carrying Out the Invention

Hereinafter, embodiments of the present invention will be described in detail.

The process of the present invention is a process for producing a compound represented by the formula (3): 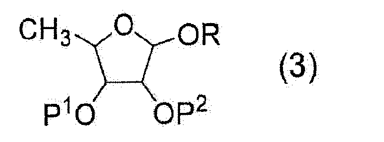 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
which comprises hydrogenating a compound represented by the formula (1) or the formula (2) in the presence of a metal catalyst: 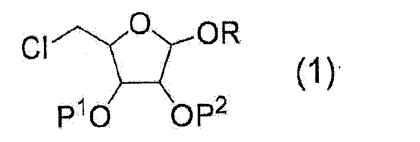 wherein P¹ and P² independently represent a hydrogen atom or an acyl group, OP¹ and OP² may together form an acetal group, and R represents a hydrogen atom, an alkyl group, an aryl group, an aralkyl group, or an acyl group; 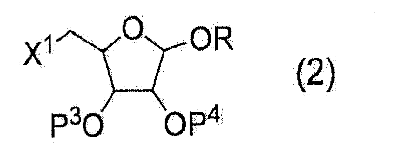 wherein X¹ represents Br or I, P³ and P⁴ independently represent a hydrogen atom or an acyl group, and R represents a hydrogen atom, an alkyl group, an aryl group, an aralkyl group, or an acyl group.

In the compounds herein defined by the formulae (1) to (5), (7), (10), and (11), the configurations of 1-, 2-, 3-, and 4-positions are not particularly limited. In addition, a sugar used in the present invention may be in a D-form, L-form, or racemic form. Such sugar is preferably ribose and more preferably ribose in a D-form.

In the compounds represented by the formulae (1) to (5), (7), (10), and (11), P¹ and P² independently represent a hydrogen atom or an acyl group, OP¹ and OP² may together form an acetal group, and P³ and P⁴ independently represent a hydrogen atom or an acyl group. Specific examples of such substituent include those described below.

Either an aliphatic acyl group or an aromatic acyl group may be used as an acyl group. For example, an acyl group with a carbon number of 1 to 20, preferably I to 10, and further preferably 1 to 7 can be used. Preferably, specific examples of such acyl group include a formyl group, an acetyl group, a propionyl group, a butyryl group, a pentanoyl group, a hexanoyl group, a heptanoyl group, an isobutyryl group, a pivaloyl group, a cyclohexane carbonyl group, a benzoyl group, a chloroacetyl group, a dichloroacetyl group, a trichloroacetyl group, a trifluoroacetyl group, and a methoxyacetyl group. Further preferred examples thereof include an acetyl group and substituted acetyl groups such as a chloroacetyl group, a dichloroacetyl group, a trichloroacetyl group, and a trifluoroacetyl group. A particularly preferred example is an acetyl group.

A cyclic acetal used may be an aliphatic acetal or an aromatic acetal. For example, an acetal with a carbon number of 1 to 20 can be used. Specific examples of an acetal include a methylene acetal, an ethylidene acetal, an acrolein acetal, a benzylidene acetal, a p-methoxybenzylidene acetal, a mesitylene acetal, an isopropylidene ketal, a cyclohexylidyne ketal, and a benzophenone ketal. Preferably, a benzylidene acetal and an isopropylidene ketal can be used.

In the compounds represented by the formulae (1) to (5), (7), (10), and (11), R represents a hydrogen atom, an alkyl group, an aryl group, an aralkyl group, or an acyl group, R¹ represents an acyl group, and R² represents an alkyl group, an aryl group, or an aralkyl group. Specific examples thereof include those described below.

Preferably, an alkyl group used is a linear, branched, or cyclic alkyl group with a carbon number of 1 to 20. More preferably, a linear, branched, or cyclic alkyl group with a carbon number of 1 to 10 is used. Further preferably, a linear, branched, or cyclic alkyl group with a carbon number of 1 to 6 is used. Examples thereof include a methyl group, an ethyl group, an isopropyl group, a normalpropyl group, a normalbutyl group, an isobutyl group, a t-butyl group, a normalhexyl group, and a cyclohexyl group. Particularly preferably, a linear or branched alkyl group with a carbon number of 1 to 3 is used. Most preferably, a methyl group is used.

Preferably, an aryl group used is a substituted or non-substituted aryl group with a carbon number of 6 to 20. Specific examples thereof include a phenyl group, a 1-naphthyl group, a 2-naphthyl group, an o-methylphenyl group, an m-methylphenyl group, a p-methylphenyl group, an o-methoxyphenyl group, an m-methoxyphenyl group, a p-methoxyphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,5-trimethylphenyl group, a 2,3,6-trimethylphenyl group, a 2,4,6-trimethylphenyl group, an o-nitrophenyl group, an m-nitrophenyl group, and a p-nitrophenyl group. Preferably, a phenyl group is used.

Preferably, an aralkyl group used is a substituted or non-substituted aralkyl group with a carbon number of 7 to 12. Examples thereof include a benzyl group, a 4-methylbenzyl group, a 4-methoxybenzyl group, and a 4-bromobenzyl group. More preferably, a benzyl group is used.

An acyl group used may be an aliphatic acyl group or an aromatic acyl group. An example thereof is an acyl group with a carbon number of 1 to 20, preferably 1 to 10, and more preferably 1 to 7. Preferably, specific examples of an acyl group include a formyl group, an acetyl group, a propionyl group, a butyryl group, a pentanoyl group, a hexanoyl group, a heptanoyl group, an isobutyryl group, a pivaloyl group, a cyclohexane carbonyl group, a benzoyl group, a chloroacetyl group, a dichloroacetyl group, a trichloroacetyl group, a trifluoroacetyl group, and a methoxyacetyl group. Further preferably, an acetyl group and a substituted acetyl group such as a chloroacetyl group, a dichloroacetyl group, a trichloroacetyl group, or a trifluoroacetyl group can be used. Particularly preferably, an acetyl group is used.

Hydrogenation in the presence of a metal catalyst can be performed by a general process. Specifically, a process that involves the use of hydrogen molecules, cyclohexadienes, a formic acid, or a hydrazine can be used. Preferably, a process that involves the use of hydrogen molecules can be used.

The term "hydrogen molecules" used in the present invention refers to a hydrogen gas that is used in general. As long as a reduction reaction proceeds as a result of hydrogenation, the purity of the hydrogen gas is not limited. However, in view of the reaction rate, higher purity is more preferable.

For example, a metal catalyst used is a sponge metal catalyst, or a transition metal catalyst supported by activated carbon or alumina. Specific examples thereof include those described below.

Examples of a sponge metal catalyst include sponge nickel prepared by dissolving a nickel-aluminium alloy in alkali, sponge cobalt prepared by dissolving a cobalt-aluminium alloy in alkali, and sponge copper prepared by dissolving a copper-aluminium alloy in alkali. Preferably, sponge nickel and sponge cobalt are used. Most preferably, sponge nickel is used. In addition, a specific example of a transition metal catalyst supported by activated carbon or alumina is a catalyst obtained by allowing activated carbon or alumina to support a transition metal belonging to any of groups 8 to 10 in the periodic table. Specific examples thereof include Ru/C, Rh/C, Pd/C, Pd-alumina, and Pt/C. Preferably, Pd/C and Pt/C can be used. A most preferable example of a metal catalyst used in the present invention is sponge nickel.

When the amount of a metal catalyst used is excessively low, it takes long time to complete a reaction or a reaction is discontinued, which is problematic. On the other hand, the use of a metal catalyst in an excessively high amount is not preferable in view of cost or aftertreatment. Therefore, the amount of metal catalyst is preferably 0.1% by weight to 1000% by weight, further preferably 1% by weight to 500% by weight, and most preferably 1% by weight to 100% by weight relative to the amount of the compound represented by the formula (I), the formula (2), or the formula (5) to be used as a starting material.

A specific process for hydrogenation in the presence of a metal catalyst is not limited as long as reaction is carried out in a hydrogen atmosphere. However, preferably, a hydrogen gas is used. Hydrogenation can be carried out at ordinary pressures or under pressurized conditions. In addition, a hydrogen gas can be introduced. However, in view of reaction time, reaction is carried out under pressurized conditions of preferably 0.1 MPa to 10 MPa, more preferably 0.1 MPa to 5 MPa, and most preferably 0.2 MPa to 1 MPa.

The reaction temperature for hydrogenation in the presence of a metal catalyst can be adequately predetermined in accordance with the boiling point of a solvent used and the upper limit temperature of a reaction system. However, it is preferably 0°C to 300°C, more preferably 10°C to 200°C, and most preferably 20°C to 120°C.

The reaction time may be 10 minutes to several days. However, in view of production cost reduction, it is preferable to terminate a reaction within preferably 48 hours and more preferably 1 to 24 hours.

Examples of a solvent used for hydrogenation in the presence of a metal catalyst include water, an alcohol-based solvent, an ether-based solvent, an aliphatic hydrocarbon-based solvent, an aromatic hydrocarbon-based solvent, an ester, a ketone-based solvent, and an amide-based solvent.

An example of an alcohol-based solvent used is alcohol having a linear, branched, or cyclic alkyl group with a carbon number of 1 to 20. Specific examples thereof include methanol, ethanol, 1-propanol, 2-propanol, 1-butanol, 2-butanol, t-butanol, t-amylalcohol, 1-hexanoyl, 1-heptanal, cyclohexanol, and methylcyclohexanol. Such an alcohol-based solvent is preferably alcohol having a linear, branched, or cyclic alkyl group with a carbon number of 1 to 10 and more preferably alcohol having a linear of branched alkyl group with a carbon number of 1 to 5.

An example of an ether-based solvent is a linear or cyclic ether. Specific examples thereof include di-normal-butyl ether, methyl cyclopentyl ether, tetrahydrofuran, tetrahydropyran, and dioxane. Such an ether-based solvent is preferably tetrahydrofuran, tetrahydropyran, or dioxane.

Examples of an aliphatic or aromatic hydrocarbon-based solvent include heptane, toluene, and xylene. Preferably, toluene is used.

Examples of an ester or ketone-based solvent include ethyl acetate, butyl acetate, methyl butyrate, ethyl butyrate, methyl ethyl ketone, diethyl ketone, and methyl isobutyl ketone. Preferably, ethyl acetate, isopropyl acetate, methyl ethyl ketone, and methyl isobutyl ketone are used,

Examples of an amide-based solvent include N-methyl-2-pyrrolidinone and N, N-dimethylformamide. Preferably, N, N-dimethylformamide is used.

In the above reaction, the solvents can be used alone or, if necessary, in the form of a mixed solvent.

Preferably, a reaction solvent used for hydrogenation in the present invention is water, an alcohol-based solvent, or an ester-based solvent in view of availability in industrial-scale practice and high reaction yield. Further preferably, it is water or an alcohol-based solvent. Most preferably, it is methanol, 2-propanol, 1-propanol, 2-butanol; t-butanol, or t-amylalcohol.

When the reaction is carried out with the use of a mixed solvent, a combination of an alcohol-based solvent and an ether-based solvent and a combination of an alcohol-based solvent and an aromatic hydrocarbon-based solvent are preferable. More specifically, a combination of an alcohol-based solvent with a carbon number of 1 to 10 and an ether-based solvent and a combination of an alcohol-based solvent with a carbon number of 1 to 10 and an aromatic hydrocarbon-based solvent are used. Preferably, a combination of an alcohol-based solvent with a carbon number of 1 to 5 and an ether-based solvent and a combination of an alcohol-based solvent with a carbon number of 1 to 5 and an aromatic hydrocarbon-based solvent are used. More preferably, a combination of 2-propanol and an ether-based solvent and a combination of 2-propanol and an aromatic hydrocarbon are used. Most preferably, a combination of 2-propanol and tetrahydrofuran and a combination of 2-propanol and toluene are used.

The lower limit amount of a solvent used for hydrogenation in the presence of a metal catalyst is not particularly limited. On the other hand, the use of solvent in an excessive amount is not preferable in view of cost or aftertreatment. Therefor, in view of the volume of a reaction vessel and operability, the amount (in terms of volume) of solvent used is 0.1 to 100 times, preferably 1 to 50 times, and further preferably 2 to 30 times greater than the amount (in terms of weight) of a compound of the formula (1), (2), or (5) used as a starting material. The density of solvent used is not particularly limited. However, it is 0.7 to 1.5 g/cm³, preferably 0.8 to 1.3 g/cm³, and further preferably 0.8 to 1.1 g/cm³ at ordinary temperatures.

Hydrogenation in the presence of a metal catalyst can be performed without the addition of bases. However, in order to capture acids generated as by-products along with the progress in reduction, it is preferable to perform hydrogenation in the presence of bases.

Organic bases such as triethylamine, diethylamine, ethylamine, diisopropylamine, N,N-diisopropylethylamine, 1, 8-diazabicyclo[5.4.0]undec-7-ene (DBU), and pyridine may be used as bases. Examples of bases that can be used include: alkali metal hydroxides such as sodium hydroxide and potassium hydroxide; phosphates such as sodium phosphate, potassium phosphate, and calcium phosphate; carbonates such as lithium carbonate, sodium carbonate, potassium carbonate, magnesium carbonate, calcium carbonate, barium carbonate, and ammonium carbonate; hydrogen carbonates such as sodium hydrogen carbonate, potassium hydrogen carbonate, and ammonium hydrogen carbonate; and inorganic bases such as ammonia. Desirably, triethylamine and DBU are used as organic bases. Further preferably, carbonates such as lithium carbonate, sodium carbonate, magnesium carbonate, calcium carbonate, and barium carbonate are used as inorganic bases.

The amount of base used is not limited as long as reaction proceeds. On the other hand, the use of the same in an excessive amount is not preferable in view of cost or aftertreatment. Therefore, the ratio of the mole of a base to the amount of substance (mole) of a substrate that is reduced via hydrogenation is 0.5:1 to 10:1, more preferably 1:1 to 5:1, and most preferably 1.2:1 to 2:1:

For hydrogenation in the presence of a metal catalyst, an additive such as a halogenated alkali metal salt may be used. Specific examples thereof include LiI, LiBr, NaI, NaBr, KI, and KBr. Preferably, LiI, NaI, and KI are used. The ratio of the amount (mole) of such additive used to the amount of a compound of the formula (1), (2), or (5) used as a starting material is 0.01:1 to 10:1, more preferably 0.1:1 to 5:1, and further preferably 0.2:1 1 to 2:1.

The compound of the formula (5) used in the present invention can be produced by reacting the compound represented by the formula (4): 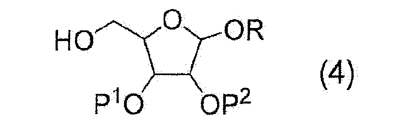 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
with an acid halide or a halogenated salt of an acid halide and an alkali metal and subjecting the resultant to acid or alkali treatment;

As an acid halide, an acid chloride or an acid bromide such as POCl₃, COCl₂, (COCl)₂, SO₂Cl₂, SOCl₂, or SOBr₂, p-toluenesulfonyl chloride, or methanesulfonyl chloride can be used. In view of good availability, preferably, POCl₃, SOCl₂, or SOBr₂ is used. Most preferably, SOCl₂ is used.

The use of an acid halide in an excessive amount is not preferable in view of cost or aftertreatment. Therefore, the ratio of the mole of an acid halide used to the amount of substance (mole) of the compound represented by the formula (4) is preferably 1:1 to 20:1 and more preferably 2:1 to 10:1.

In the present invention, an acid halide may be used alone. Alternatively, an acid halide may be used in combination with a halogenated alkali metal salt. In such case, LiI, LiBr, NaI, NaBr, KI, KBr, or the like can be used as a halogenated alkali metal salt. In view of reactivity in the hydrogenation following the above reaction, it is desirable to use an iodine compound. Preferably, LiI; NaI, or KI is used as a halogenated alkali metal salt. The ratio of the mole of a halogenated alkali metal salt used to the amount of substance (mole) of the compound represented by the formula (4) used as a starting material is generally 1:1 to 10:1, more preferably 1.2:1 to 5:1, and further preferably 1.5:1 1 to 2:1.

When a compound of the formula (5) is produced by allowing an acid halide and a halogenated alkali metal salt to act on a compound of the formula (4), a base can be used. The presence or absence of a base is not limited. However, it is preferable to use a base because a compound of the formula (5) can be obtained at a higher yield by capturing acids generated as by-products. Examples of a base that can be used include organic bases such as triethylamine, diethylamine, ethylamine, diisopropylamine, N,N-diisopropylethylamine, and pyridine; alkali metals hydroxides such as sodium hydroxide and potassium hydroxide; carbonates such as sodium carbonate and potassium carbonate; inorganic bases such as hydrogen carbonates, including sodium hydrogen carbonate and potassium hydrogen carbonate. Such base is preferably an organic base and more preferably triethylamine or pyridine such that a compound of the formula (5) can be obtained at a higher yield.

The use of such base in an excessive amount is not preferable in view of cost or aftertreatment. Therefore, the ratio of the mole of base used to the amount of substance (mole) of the compound represented by the formula (4) used as a starting material is preferably 1;1 to 20:1 and more preferably 2:1 to 10:1.

Examples of a solvent used for producing a compound represented by the formula (5) include: nitrile-based solvents such as acetonitrile and benzonitrile; ether-based solvents such as di-normal-butyl ether, di-normal-propyl ether, tetrahydrofuran, and tetrahydropyran; aromatic hydrocarbon-based solvents such as toluene and xylene; and organic bases such as pyridine and triethyamine. Such solvent is preferably a nitrile-based solvent, an ether-based solvent, or an organic base, more preferably acetonitrile, tetrahydrofuran, pyridine, triethylamine, and most preferably acetonitrile. The amount of solvent is not limited as long as a compound in a reaction vessel can be sufficiently agitated. However, the use of such solvent in an excessive amount is not preferable in view of cost or aftertreatment. The amount (in terms of volume) of solvent is preferably 1 to 30 times, more preferably 2 to 15 times, and most preferably 3 to 10 times greater than the amount (in terms of weight) of the compound of the formula (4) used as a starting material.

In the present invention, after reacting an acid halide and a halogenated alkali metal salt with a compound of the formula (4) in the presence of an organic base, it is possible to add an operation of filtering off a hydrochloride of a tertiary amine or a nitrogen-containing heterocyclic compound, such as a triethylamine hydrochloride or a pyridine hydrochloride.

As long as a compound of the formula (5) can be obtained, an operation of filtering off such an addition salt is not limited. Specifically, a funnel and filter paper can be used. Alternately, a filter press may be used for filtration. In order to achieve a higher yield, a solvent is sprinkled over a residue so as to filter off an addition salt of an amine or a heterocyclic compound, followed by removal of the solvent from the filtrate by distillation under pressurized conditions. Thus, a product of interest can be obtained. Alternatively, the residue is suspended in a solvent and then filtration can be carried out.

The temperature for producing a compound represented by the formula (5) is preferably 0°C to 1000°C and more preferably 10°C to 80°C. The reaction time may be 1 hour to several days. In order to reduce the production cost, the reaction is terminated preferably within 24 hours and more preferably 1 to 12 hours. The reaction can be carried out at ordinary pressures or in the air. Also, the reaction can be performed under pressurized conditions in an inert gas such as nitrogen or argon according to need.

In the present invention, a compound of the formula (5) can be produced by reacting acid halide and a halogenated alkali metal salt with a compound of the formula (4), followed by acid or alkali treatment.

When acid treatment is performed, either a weak acid or a strong acid can be used. However, preferably, a strong acid is used. In addition, examples of an acid that can be used include: inorganic acids such as sulfuric acid, hydrochloric acid, and nitric acid; and organic acids such as formic acid, methanesulfonic acid, and p-toluenesulfonic acid. Preferably, an inorganic acid is used. More preferably, sulfuric acid is used.

When alkali treatment is performed, either a weak alkali or a strong alkali can be used. In addition, examples of an alkali include: inorganic bases such as sodium hydroxide, potassium hydroxide, magnesium hydroxide, calcium hydroxide, sodium carbonate, potassium carbonate, sodium hydrogen carbonate, potassium hydrogen carbonate, and ammonia; and organic bases such as triethylamine and pyridine.

The above examples of alkali, including inorganic bases and organic bases, may be used alone or may be added to water or an alcohol-based solvent for use.

When an alkali is used, it is preferable to use an aqueous solution containing an inorganic base, an alcohol, solution containing an inorganic base, ammonia water, or an alcohol solution containing ammonia. It is more preferable to use an aqueous solution containing potassium carbonate, ammonia water, or an alcohol solution containing ammonia.

As long as the reaction can proceed, the use of an acid or an alkali is not limited. However, it is preferable to use an alkali. An example of an alcohol used herein is alcohol having a linear, branched, or cyclic alkyl group with a carbon number of 1 to 10. Specific examples thereof include methanol, ethanol, 1-propanol, 2-propanol, 1-butanol, 2-butanol, t-butanol, 1-hexanoyl, 1-heptanol, cyclohexanol, and methylcyclohexanol. Preferably, methanol, ethanol, 2-propanol, and 1-butanol are used. Preferably, in order to allow the reaction to proceed at a higher rate, the concentration of an aqueous solution containing an inorganic base, an aqueous solution containing ammonia, or an alcohol solution containing ammonia is set to a higher level.

A compound represented by the formula (5) can be separated by a process comprising concentrating a reaction solution or an extraction operation with the use of a solvent. However, it is preferable to carry out an extraction operation with the use of a solvent such that a compound of the formula (5) with a higher purity can be obtained. Examples of a solvent used include: ester-based solvents such as ethyl acetate and butyl acetate; aromatic hydrocarbon-based solvents such as toluene and xylene; a linear, branched, or cyclic alcohol-based solvent with a carbon number of 4 to 6 such as 1-butanol, 2-butanol, and hexanol; ether-based solvents such as dibutyl ether, diisopropyl ether, tetrahydrofuran, and tetrahydropyran; and acetonitrile. Preferably, ester-based solvents such as ethyl acetate and butyl acetate and ether-based solvents such as dibutyl ether, diisopropyl ether, tetrahydrofuran, and tetrahydropyran are used. More preferably, ethyl acetate and tetrahydrofuran are used. Further preferably, ethyl acetate is used.

Preferably, a solvent is used in a greater amount in view of extraction efficiency. Meanwhile, preferably, a solvent is used in a smaller amount in view of operability and economic efficiency. The amount (in terms of volume) of solvent used is preferably 1 to 20 times and more preferably 2 to 10 times greater than the amount (in terms of weight) of a starting material represented by the formula (4).

Regarding a compound of the formula (5), a β-anomer thereof is a solid substance and an α-anomer thereof is an oily substance. Therefore, the compound can be separated from by-products generated during conversion from ribose via crystallization or washing by suspension. Accordingly, a β-anomer of a compound represented by the formulae (5) can be isolated at a higher purity.

In the crystallization operation, a reaction product containing a compound of the formula (5) is suspended in a solvent, followed by heating. The resulting solution is cooled to an ice temperature, for example, followed by filtration. Thus, the resultant in a crystal form can be obtained. In the operation of washing by suspension, a reaction product containing a compound of the formula (5) is suspended in a solvent, followed by agitation and then filtration. Accordingly, a β-anomer in a crystal form can be obtained.

Example of a solvent used for crystallization or washing by suspension include: aliphatic hydrocarbon-based solvents such as pentane, hexane, and heptane; aromatic hydrocarbon-based solvents such as benzene, toluene, and xylene; ester-based solvents such as ethyl acetate and isopropyl acetate; alcohol-based solvents such as methanol, and isopropanol; and ether-based solvents such as diethyl ether, diisopropyl ether, dibutyl ether, and tetrahydrofuran. These solvents may be used alone or in combination. Preferably, aliphatic hydrocarbon-based solvents such as pentane, hexane, and heptane and aromatic hydrocarbon-based solvents such as benzene, toluene, and xylene are used. More preferably, toluene and heptane are used. Further preferably, toluene is used. When a solvent is used in an excessively small amount, the purity of β-anomer decreases due to incorporation of impurities, which is problematic. Meanwhile, the use of such solvent in an excessive amount is not preferable in view of cost or aftertreatment. The amount (in terms of volume) of solvent used is preferably 0.1 to 10 times, more preferably 0.5 to 5 times, and most preferably 1 to 3 times greater than the amount (in terms of weight) of a compound represented by the formula (5).

A compound of the formula (5) can be purified with the use of an adsorbent such as silica gel, activated carbon, activated clay, ion-exchange resin, or Celite. Examples of a treatment process include a process wherein a solution containing a compound represented by the formula (5) is passed through a column tube filled with such an adsorbent with the use of a solvent or the like and a process wherein an adsorbent is added to a solution or suspension containing a compound represented by the formula (5), the mixture is agitated to cause adsorption of impurities, and then the adsorbent is filtered off. Preferably, silica gel is used such that a compound of the formula (5) with a higher purity can be obtained. However, in view of economic efficiency, it is preferable to use activated carbon or activated clay. Regarding a treatment process, it is preferable to use the above process wherein an adsorbent is filtered off after suspension in a solvent in view of operability. The type of activated carbon or activated clay is not limited as long as the purity of compound represented by the formula (5) subjected to filtering-off treatment can be improved. The weight ratio of the amount of adsorbent used to the amount (in terms of weight) of a compound of the formula (5) is preferably 0.001:1 to 10:1, more preferably 0.01:1 to 5:1, and most preferably 0.05:1 to 1:1.

As a result of purification of a compound of the formula (5) with the use of an adsorbent such as silica gel, activated carbon, activated clay, ion-exchange resin, or Celite, the content of sulfur component contained in a compound of the formula (5) can be reduced. A sulfur component is toxic to a metal catalyst upon hydrogenation of a compound of the formula (5) in the presence of a metal catalyst, resulting in inhibition of, the hydrogenation reaction, which is problematic. Therefore, it is more preferable for the compound to contain a sulfur component at a lower content. The content of sulfur component at which the progress of reaction is not inhibited is preferably 0.01% to 1% by weight, more preferably 0.05% to 0.5% by weight, and further preferably 0.1% to 0.3% by weight relative to the weight of a compound represented by the formula (5).

The compound of the formula (5) purified as above can be subjected to the subsequent step of hydrogenation in the presence of a metal catalyst. In addition, after acetylation of a hydroxyl group in a compound represented by the formula (5), the compound can be subjected to hydrogenation in the presence of a metal catalyst.

A compound represente by the formula (6): 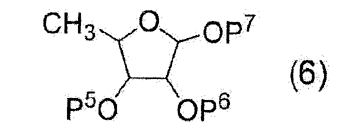 wherein P⁵, P⁶, and P⁷ independently represent an acyl group and may be the same or different;
can be produced by converting a hydroxyl group or a substituted hydroxyl group in a compound represented by the formula (3) produced by the process of the present 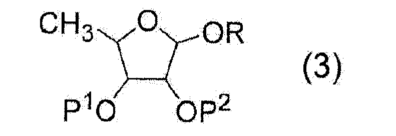 wherein P¹ and P² independently represent a hydrogen atom or an acyl group, OP¹ and OP² may together form an acetal group, R represents a hydrogen atom, an alkyl group, an aryl group, an aralkyl group, or an acyl group, provided that P¹, P², and R do not simultaneously represent an acyl group.

Similarly, a compound represented by the formula (8): 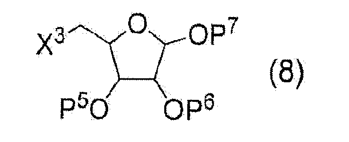 wherein, X³ represents Cl, Br, or I and P⁵, P⁶, or P⁷ independently represent an acyl group and may be the same or different; can be produced by converting a hydroxyl group or a protected hydroxyl group in a compound represented by the formula (7) produced by the process of the present invention into an acyl-protected hydroxyl group; 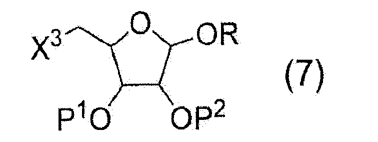 wherein, X³ represents Cl, Br, or I and P¹ and P² independently represent a hydrogen atom or an acyl group, OP¹ and OP² may together form an acetal group, and R represents a hydrogen atom, alkyl group, aryl group, aralkyl group, or acyl group, provided that P¹, P², and R do not simultaneously represent an acyl group.

In the compounds of the formulae (6) and (8) defined herein, the configurations of 1-, 2-, 3-, and 4-positions are not particularly limited. In addition, a sugar used in the present invention may be a D-form, an L-form, or a racemic form. Preferably, ribose is used. More preferably, ribose in a D-form is used.

In the compounds of the formulae (6) and (8), P⁵, P⁶, and P⁷ independently represent an acyl group and may be the same or different. Specific examples of such substituent are described below.

As an acyl group, either an aliphatic acyl group or an aromatic acyl group may be used. An example thereof is an acyl group with a carbon number of 1 to 20, preferably 1 to 10, and more preferably 1 to 7. Preferably, specific examples of an acyl group include a formyl group, an acetyl group, a propionyl group, a butyryl group, a pentanoyl group, a hexanoyl group, a heptanoyl group, an isobutyryl group, a pivaloyl group, a cyclohexane carbonyl group, a benzoyl group, a chloroacetyl group, a dichloroacetyl group, a trichloroacetyl group, a trifluoroacetyl group, and a methoxyacetyl group. More preferably, an acetyl group and a substituted acetyl group such as a chloroacetyl group, a dichloroacetyl group, a trichloroacetyl group, or a trifluoroacetyl group are used. Particularly preferably, an acetyl group is used.

In a reaction in which a compound of the formula (3) is converted into a compound of the formula (6) and in a reaction in which a compound of the formula (7) is converted into a compound of the formula (8), it is first necessary to carry out deprotection, that is to say, an operation that allows P¹, P², or R to represent a hydrogen atom in a case in which either one of or both of P¹ and P² among P¹, P², and R that represent a substituted hydroxyl group in a compound represented by the formula (3) or (7) represent(s) an acyl group, which is a non-acetyl group (Ac group), in which P¹ and P² may together form a cyclic acetal group, or in which R represents an acyl group, which is a non-acetyl group. The above deprotection process is known to those skilled in the art. For instance, the process is described in Protective Groups in Organic Synthesis, John & Wiley & Sons Inc. (1998).

For example, regarding reaction conditions for deprotection, deprotection is carried out through a reaction with an alcohol-based solvent such as methanol and water in the presence of an inorganic base such as sodium hydroxide or potassium hydroxide or in the presence of an organic base such as triethylamine or trimethylamine. As long as deprotection smoothly proceeds, the deprotection process is not limited. However, in view of economic efficiency, preferably, a deprotection process is carried out in the pretence of an inorganic base.

A compound of the formula (3) or (7) is subjected to a step of conversion into a compound in which P¹ and P² are both acetylated or a step of acetylation for conversion into a compound of the formula (6) or (8) after deprotection of hydroxyl groups at 2- and 3-positions.

In a reaction in which a compound of the formula (3) is converted into a compound of the formula (6) and in a reaction in which a compound of the formula (7) is converted into a compound of the formula (8), a step of acetylation for conversion into a compound of the formula (6) or (8) is carried out in a case in which either one of or both of P¹ and P² among P¹_{,} P², and R that represent a hydroxyl group or a substituted hydroxyl group in a compound represented by the formula (3) or (7) represent(s) hydroxyl group or acetyl group, or in which R represents an alkyl group, an aralkyl group, or an aryl group (provided that P¹, P², and R do not simultaneously represent an acetyl group).

An acetylation process in a case in which either one of or both of P¹ and P² in a compound represented by the formula (3) or (7) represent(s) a hydroxyl group is described in the aforementioned Protective Groups in Organic Synthesis, John & Wiley & Sons Inc. (1998) or the like. For example, acetylation can be carried out by allowing an acetylation agent such as acetic anhydride or acetyl chloride to act in the presence of an organic base such as pyridine or triethylamine or in the presence of an inorganic base such as sodium acetate or potassium acetate. After acetylation, a 2,3-diacetyl form in which P¹ and P² are both acetylated is obtained as a temporal product by carrying out isolation/purification via a concentration operation, solvent extraction, or the like. Thus, it is possible to proceed to a step of converting a substituent R of a composition of the formula (3) or (7) into a substituent of a composition of the formula (6) or (8) (hereinafter referred to as acetolysis) in a stepwise manner. It is also possible to induce acetolysis without isolation/purification of a 2,3-diacety form. As long as acetylation proceeds, reaction conditions are not limited. However, in view of ease of operability, it is preferable to immediately proceed to acetolysis without isolation/purification.

Regarding conditions of acetolysis, for example, an acetylation agent is allowed to act in the presence of an acid. Examples of acid that can be used for acetolysis include an inorganic acid such as sulfuric acid or hydrochloric acid and an organic acid such as p-toluenesulfonic acid, methanesulfonic acid, or formic acid. However, an inorganic acid is preferable because it is less expensive. More preferably, it is preferable to use sulfuric acid.

The amount of acid used is not particularly limited as long as acetolysis of an alkoxy group at 1-position proceeds. For example, the ratio of the mole of acid to the amount of substance (mole) of a compound of the formula (3) or (7) is preferably 3:1 or less. In view of ease of neutralization operation during aftertreatment, the mole ratio is preferably 1:1 or less. Examples of an acylation agent used for acetolysis include acetic anhydride, acetyl chloride, and acetic acid. These may be used alone or in combination. A combination of acetic anhydride and acetic acid is preferable such that a higher yield can be achieved.

In addition, a base is further added upon acetolysis. Examples of a base that can be used include: organic bases such as amines (e.g., trimethylamine, triethylamine, and N,N-diisopropylethylamine) and pyridine; and inorganic bases such as potassium carbonate, sodium carbonate, and sodium hydrogen carbonate. Preferably, an organic base is used such that a higher yield can be achieved. The amount of base used is not particularly limited. However, the ratio of the mole of base to the amount of substance (mole) of a compound of the formula (3) or (7) is preferably 3:1 or less, more preferably 2:1 or less, and most preferably 1:1 or less.

After acetolysis, a compound of the formula (6) or (8) can be obtained by a concentration or extraction operation. An isolation/purification process is not particularly limited. Either a concentration operation or an extraction operation or a combination of both operations may be carried out. In view of stability of a compound of the formula (6) or (8), reagents used for a reaction can be removed by carrying out an extraction operation. Therefore, an extraction operation is preferably used. Examples of a solvent used for an extraction operation include: ester-based solvents such as methyl acetate and ethyl acetate; aromatic hydrocarbon-based solvents such as toluene and xylene; and ether-based solvents such as diethyl ether and tetrahydrofuran. In view of extraction efficiency and economic efficiency, preferably, an ester-based solvent and an aromatic hydrocarbon-based solvent are used. More preferably, ethyl acetate and toluene are used. An organic layer obtained via extraction is concentrated. Then, it can be purified by silica gel column chromatography, distillation, crystallization, or the like. As long as a compound of the formula (6) or (8) can be obtained at a high purity, a purification process is not limited. However, in view of operability and economic efficiency, preferably, purification can be carried out by crystallization.

Among the compounds produced by the process of the present invention, 1,2,3-tri-O-acetyl-5-deoxy-β-D-ribofuranose can be converted into Capecitabine, which is useful as an anticancer agent, via condensation with 5-fluorocytosine with the use of HMDS (hexamethyldisilazane), followed by derivatization, by the process described in Bioorganic & Medicinal Chemistry, 2000, vol. 8, no. 8, pp. 1967-1706 or the like. Therefore, it is a compound useful as a pharmaceutical or agricultural intermediate.

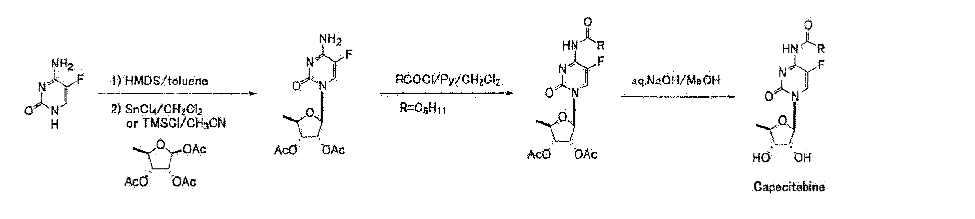

The present invention further provides the following processes:
a process for producing a mixture containing an α-anomer and a β-anomer of a compound of the formula (10): 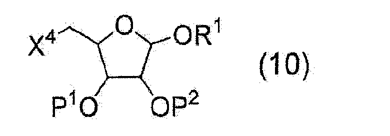 wherein X⁴ represents Cl, Br, I, r a hydrogen atom, P¹ and P² independently represent a hydrogen atom or acyl group, OP¹ and OP² may together form an acetal group, and R¹ represents an acyl group;
   in which the proportion of the β-anomer in the mixture after treatment becomes greater than that in the mixture before treatment, which comprises treating a mixture containing a compound of said formula (10) with an α-configuration at 1-position (α-anomer) and a compound of said formula (10) with a β-configuration at 1-position (β-anomer) in the presence of an acid and a poor solvent: and
   a process for producing a compound of the formula (10); 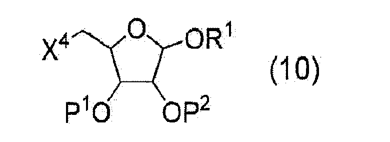 wherein X⁴ represents Cl, Br, I, or a hydrogen atom, P¹ and P² independently represent a hydrogen atom or an acyl group, OP¹ and OP² may together form an acetal group, and R¹ represents an acyl group;
   which comprises a step of allowing an acylation agent to act on a mixture containing a compound of the formula (11): 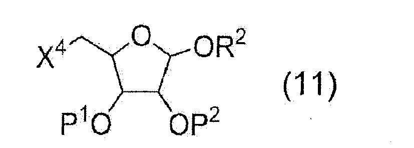
wherein X⁴ represents Cl, Br, I, or a hydrogen atom, P¹ and P² independently represent a hydrogen atom or an acyl group, OP¹ and OP² may together form an acetal group, and R² represents an alkyl group, an aryl group, or an aralkyl group;
with an a-configuration at 1-position (α-anomer) and a compound of said formula (11) with a β-configuration at 1-position (β-anomer) in the presence of an acid and a poor solvent before reaction so as to obtain a mixture containing a compound of said formula (10) with an α-configuration at 1-position (α-anomer) and a compound of said formula
(10) with a β-configuration at 1-position (β-anomer) after reaction, wherein the proportion of β-anomer in the mixture after reaction becomes greater than that in the mixture before reaction. Hereinafter, the above two processes are collectively referred to as "process for increasing the proportion of β-anomer of the present invention."

Specific examples of an acyl group represented by P¹ and P² in the formulae (10) and (11), an acyl group represented by R¹ in the formula (10), an alkyl group, an aryl group, or an aralkyl group represented by R² in the formula (11) are the same as specific examples described for an acyl group represented by P¹ and P² and an acyl group represented by R¹ in the formulae (1) to (5) and (7).

In the present invention, the mole ratio of an α-anomer and a β-anomer (α-anomer : β-anomer) in a mixture in the presence of an acid and a poor solvent before treatment is preferably 100:0 to 20:80, more preferably 80:20 to 25:75, and further preferably 50:50 to 25:75.

In the present invention, the mole ratio of an α-anomer and a β-anomer (α-anomer : β-anomer) in a mixture in the presence of an acid and a poor solvent after treatment is preferably 30:70 to 0:100, more preferably 20:80 to 0:100, further preferably 15:85 to 0:100, and particularly preferably 10:90 to 0:100. More specifically, when 1,2,3-tri-O-acetyl-5-deoxy-β-D-ribofuranose is synthesized, the mole ratio of the produced α-anomer and the produced β-anomer α-anomer : β-anomer) is preferably 30:70 to 0:100, more preferably 20:80 to 0:100, further preferably 15:85 to 0:100, and particularly preferably 10:90 to 0:100. In addition, when 1,2,3-tri-O-acetyl-5-deoxy-5-chloro-β-D-ribofuranose is synthesized, the product ratio of an α-anomer to a β-anomer (α-anomer : β-anomer) is preferably 30:70 to 0:100, more preferably 20:80 to 0:100, further preferably 15:85 to 0:100, and particularly preferably 10:90 to 0:100.

Acid used for a process for increasing the β-anomer proportion in the present invention may be a weak acid or a strong acid. However, it is preferably strong acid. In addition, such acid may be an inorganic acid (e.g., sulfuric acid, hydrochloric acid, and nitric acid) or an organic acid (e.g., formic acid, benzoic acid, methanesulfonic acid, trifluoromethanesulfonic acid, and p-toluenesulfonic acid). However, it is preferably an inorganic acid. As such acid, it is particularly preferable to use sulfuric acid or hydrochloric acid.

The amount of an acid used is not particularly limited as long as the β-anomer proportion in a compound of the formula (10) can be increased. For instance, the ratio of the mole of an acid to the amount of a substance (mole) of a compound of the formula (10) or (11) used as a starting material is preferably 5:1 or less and more preferably 3:1 or less.

In a process for increasing the β-anomer proportion of the present invention, a poor solvent is used. A poor solvent may exist at the beginning of reaction. Alternatively, it may be added during reaction. It is also possible to add a poor solvent before the termination of reaction so as to cause the β-anomer to precipitate. As a poor solvent that can be used in the present invention, a solvent in which the solubility of a compound of the formula (10) or (11) used as a starting material is low can be used. For instance, according to the present invention, a solvent that can be used as a poor solvent is a solvent in which the solubility of a compound of the formula (10) or (11) becomes preferably 200 g/L or less, more preferably 100 g/L or less, and further preferably 20 g/L or less.

Preferably, a poor solvent used in the present invention may be an ester-based solvent, an ether-based solvent, an aliphatic hydrocarbon-based solvent, or an aromatic hydrocarbon-based solvent. Examples of an ester-based solvent include ethyl acetate and butyl acetate. Examples of an ether-based solvent include diethyl ether, diisopropyl ether, di-normal-propyl ether, di-normal-butyl ether, methyl isopropyl ether, methyl-t-butyl ether, ethyl-t-butyl ether, cyclopentyl methyl ether, tetrahydrofuran, tetrahydropyran, and dioxane. Examples of an aliphatic hydrocarbon-based solvent include pentane, hexane, and heptane. Examples of an aromatic hydrocarbon-based solvent include benzene, toluene, and xylene. Preferably, an ether-based solvent, an aliphatic hydrocarbon-based solvent, and an aromatic hydrocarbon-based solvent are used, but not limited thereto, A poor solvent may be used alone or in combination of a plurality of mixed solvents.

The amount of a poor solvent used is not particularly limited as long as a compound of the formula (10) can be produced by allowing an acylation agent to act on a compound of the formula (11). However, for instance, the amount thereof is preferably not more than 50 times, more preferably not more than 20 times, and particularly preferably not more than 10 times greater than the amount (in terms of weight) of a compound represented by the formula (11).

Further, in the process for increasing the β-anomer proportion of the present invention, a dehydration agent is allowed to exist during treatment in the presence of an acid and a poor solvent. Examples of a dehydration agent that can be used in the present invention include: dehydration agents used in a dehydration process involving water adsorption (e.g., molecular sieve, anhydrous sodium sulfate, anhydrous magnesium sulfate, anhydrous calcium chloride); and dehydration agents used in a dehydration process based on chemical change of water (e.g., aliphatic monocarboxylic anhydrides such as acetic anhydride and propionic anhydride; aromatic monocarboxylic anhydrides such as benzoic anhydride; aliphatic polycarboxylic anhydrides such as succinic anhydride and maleic anhydride; polycyclic polycarboxylic anhydrides such as tetrahydrophthalic anhydride and hexahydrophthalic anhydride; aromatic polycarboxylic anhydrides such as phthalic anhydride and tetrabromophthalic anhydride; and acetyl chloride). Such a dehydration agent may be used in an amount that can cause removal of water contained in a reaction system. For example, the ratio of the mole of dehydration agent used to the amount of substance (mole) of a reaction component (substrate) is generally approximately 0.0001:1 to 1:1, preferably approximately 0.001:1 to 05:1, and further preferably approximately 0.01:1 to 0.1:1. Particularly, in the step of increasing the β-anomer proportion of tri-O-acetyl-5-deoxy-D-ribofuranose, it is possible to capture water contained in a solvent, raw material and reagents by adding a dehydration agent such as acetic anhydride. As a result of capture of water, side reaction is suppressed such that a β-anomer of tri-O-aeetyl-5-deoxy-D-ribofuranose can be obtained at a high yield.

An acylation agent used in the process for increasing the β-anomer proportion in the present invention is not particularly limited as long as a compound represented by the formula (10) can be produced by allowing a compound of the formula (11) to act in the presence of an acid and a poor solvent. Preferably, it is an acid halide or an acid anhydride. Specific examples of an acid halide or an acid anhydride include, but are not particularly limited to, acid chlorides such as acetyl chloride, isobutyrate chloride, pivaloyl chloride, cyclohexane carbonyl chloride, benzoyl chloride, and 4-methoxybenzoyl chloride; acid bromides such as acetyl bromide, isopropionic acid bromide, pivaloyl bromide, cyclohexane carbonyl bromide, benzoyl bromide, and 4-methoxybenzoyl bromide; and acid iodides such as acetyl iodide, isobutyrate iodide, pivaloyl iodide, cyclohexane carbonyl iodide, benzoyl iodide, and 4-methoxybenzoyl iodide. Examples of acid anhydride include acetic anhydride, propionic anhydride, pivalic anhydride, cyclohexanecarboxylic anhydride, and benzoic acid anhydride. Preferably, acetic anhydride is used. In addition, acetic acid can be used as an acylation agent. Particularly preferable examples of an acylation agent used in the present invention include acetic acid, acetic anhydride, and a mixture thereof.

Preferably, the amount of an acylation agent used is predetermined such that a β-anomer of a compound represented by the formula (10) produced by the process of the present invention precipitates. For instance, the ratio of the mole of acylation agent to the amount of substance (mole) of a compound of the formula (11) is preferably 4:1 or less and more preferably 3:1 or less. When acetic anhydride is used as an acylation agent, the ratio of the mole of acetic anhydride to the amount of substance (mole) of a compound of the formula (11) is preferably 3:1 or less.

Further, according to the process for increasing the β-anomer proportion of the present invention, a base is allowed to exist during treatment in the presence of an acid and a poor solvent or when an acylation agent is allowed to act in the presence of an acid and a poor solvent. As a base, an organic base (e.g., tertiary amine such as trimethylamine, triethylamine, N, N-diisopropylethylamine, or tri-normal-propylamine; or aromatic amine such as pyridine) or an inorganic base (e.g., potassium hydroxide or sodium hydroxide) may be used. Preferably, an organic base is used. The base used is preferably triethylamine or pyridine and more preferably pyridine.

The amount of base used is not particularly limited as long as the β-anomer proportion in a compound represented by the formula (10) can be increased. However, for example, the ratio of the mole of base to the amount of substance (mole) of a compound of the formula (10) or (11) used as a starting material is preferably 3:1 or less and more preferably 1:1 or less.

In the process for increasing the β-anomer proportion of the present invention, the treatment or reaction temperature for preparing a compound with an increased β-anomer proportion is not particularly limited. However, the temperature is preferably determined such that a β-anomer of a compound represented by the formula (10) to be generated precipitates. For instance, in the present invention, the temperature is preferably approximately -78°C to 50°C, more preferably approximately -40°C to 30°C, and further preferably approximately -20°C to 10°C. The above reaction can be carried out at ordinary pressure in the air. It is not particularly necessary to carry out the reaction in a nitrogen atmosphere. However, if necessary, the reaction can be carried out in an inert gas such as nitrogen, helium, argon, etc. under pressurized conditions.

It is possible to set the time for treatment or reaction time to 1 minute to several days. However, in view of the reduction of production cost, treatment or reaction is completed within preferably 24 hours, more preferably 5 minute to 12 hours, and further preferably 10 minutes to 5 hours.

A mixture of an α-anomer and a β-anomer of the compound represented by the formula (10) produced by the process for increasing the β-anomer proportion of the present invention described above is further purified such that a β-anomer of the compound represented by the formula (10) can be isolated. A purification process is not particularly limited, However, for example, purification can be performed by crystallization or washing by suspension. In a crystallization operation, a reaction product containing a compound of the formula (10) is suspended in a solvent, a solution obtained by heating to reflux is cooled to, for example, an ice temperature, followed by filtration. Thus, crystals can be obtained. In a washing by suspension operation, a reaction product containing a compound of the formula (10) is suspended in a solvent, followed by agitation and then filtration. Thus, crystals can be obtained. Examples of a solvent used for crystallization or washing by suspension include an alcohol-based or ether-based solvent or a mixture of such solvent and water. Preferably, as an alcohol-based solvent, methanol, ethanol, normal propyl alcohol, isopropyl alcohol, or normal butanol is used. As an ether-based solvent, preferably diethyl ether, diisopropyl ether, di-normal-propyl ether, di-normal-butyl ether, methyl isopropyl ether, methyl-t-butyl ether, ethyl-t-butyl ether, tetrahydrofuran, or dioxane is used.

According to the present invention, a D or L-ribofuranose derivative represented by the formula (12) is provided. 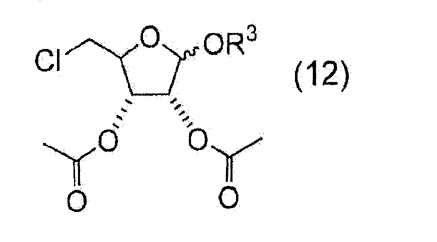 wherein a configuration of 1-position is α or β, R³ represents an alkyl group with a carbon number of 1 to 6, an aryl group with a carbon number of 6 to 20, or an aralkyl group with a carbon number of 7 to 12.

For specific examples of an alkyl group, an aryl group, and an aralkyl group represented by R³ in the formulae (12), those similar to specific examples of an alkyl group, an aryl group, and an aralkyl group represented by R in the formula formulae (1), (5), (7), (10), and (11) described herein can be used.

The D or L-ribofuranose derivative represented by the formula (12) of the present invention is a useful compound because 1,2,3-tri-O-acetyl-5-deoxyribofuranose can be produced therefrom by subjecting the derivative to a step of hydrogenation in the presence of a metal catalyst and an acetolysis step.

The present invention is hereafter described in greater detail with reference to the following examples, although the technical scope of the present invention is not limited thereto.

### Examples

### (Example 1) Synthesis of 1-O-methyl-D-ribofuranose

D-ribose (100 g, 666 mmol) and methanol (1000 mL) were introduced into a 2-L flask. Concentrated sulfuric acid (5.0 mL, 66.6 mmol) was slowly added dropwise thereto during ice cooling. The flask was heated to room temperature so as to cause a reaction at room temperature for 4 hours. Sodium acetate (16.4 g, 200 mmol) was added thereto for neutralization, followed by concentration under reduced pressure. Crude 1-O-methyl-D-ribofuranose (134 g; purity: 81%; yield: 100%) was obtained as a white turbid oily component.
[NMR data]
¹H-NMR(400MHz,D₂O-d):δ(β-anomer)3.38(s,3H),3.57-3.62(m,1H),3,76-3,80(m,1H),3.9 9-4.03(m, 2H),4.13-4.16(m,1H)4.89(d,J=1.0Hz,1H)
(α-anomer)3.42(s,3H),3.63 -3.75(m,2H),3.98-4.1 (m,3H),4.98(d,J=4.5Hz;1H)

### (Example 2A) Synthesis of 1-O-methyl-3-deoxy-5-chloro-D-ribofuranose

Crude 1-O-methyf-D-ribofuranose (134 g) synthesized in Example 1 above, acetonitrile (520 mL), and triethylamine (283 g, 2.8 mol) were introduced into a 2-L flask. Thionyl chloride (254 g, 2.1 mol) was slowly added dropwise thereto during ice cooling. Thereafter, the internal temperature was increased from 60°C to 65°C, followed by agitation with heating for 2 hours. The flask was cooled to room temperature. Then, triethylamine hydrochloride that had precipitated from the obtained reaction solution was filtered off, followed by washing with acetonitrile (300 mL). The filtrate was concentrated under reduced pressure. Ethyl acetate (700 mL) and 28% ammonia water (365 g) were added thereto so as to cause a reaction at room temperature for 1 hour. After separating the liquid into the organic layer and the aqueous layer, an extraction operation was repeated 3 times with the use of ethyl acetate (200 mL). Thus, the organic layer was separated and then introduced into a column tube filled with silica gel (100 g). The collected solution was concentrated under reduced pressure. Accordingly, crude 1-O-methyl-5-deoxy-5-chloro-D-tibofuranose (121 g; purity: 90%; yield: 89%) was obtained as a brown oily component. The sulfur content therein was not more than 0.3% by weight.
[NMR data]
¹H-NMR(400MHz,CDCl₃):δ(β-anomer)2,77(m,1H), 2.98(bs,1H),3.38(s,3H), 3.71-3.61(m,2H),4.08(m,1H),4.14(dd,J=11.9,12.1Hz,1H),4.33(m,1H),4.86(s,1H) (α-anomer)2.64(d,J=7.56Hz,1H),2.90(d,J=8.56Hz,1H),3.50(s,3H),3.70(d,J=4.32Hz,2H), 4,00-3,96(m,1H),4.23-4.13(m,2H),5.00(d,J=4.56Hz,1H)

### (Example 2B) Synthesis of 1-O-methyl-5-deoxy-5-chloro-D-ribofuranose

Crude 1-O-methyl-D-ribofuranose (134 g) synthesized in Example 1 above, acetonitrile (520 mL), and triethylamine (283 g, 2.8 mol) were introduced into a 2-L flask. Thionyl chloride (254 g, 2.1 mol) was slowly added dropwise thereto during ice cooling. Thereafter, the internal temperature was increased from 60°C to 65°C, followed by agitation with heating for 2 hours. The flask was cooled to room temperature. Then, triethylamine hydrochloride that had precipitated from the obtained reaction solution was filtered off, followed by washing with ethyl acetate (300 mL). The filtrate was concentrated under reduced pressure and ethyl acetate (700 mL) and 28% ammonia water (365 g) were added thereto so as to cause a reaction at room temperature for 1 hour. After separating the liquid into the organic layer and the aqueous layer, extraction operation was repeated 3 times with the use of ethyl acetate (200 mL). The obtained organic layer was concentrated under reduced pressure. Crude 1-O-methyl-5-deoxy-5-chloro-D-ribofuranose (129 g; purity: 85%; yield: 90%; sulfur content: 1.1% by weight) was obtained as a brown oily component. Toluene (330 mL) was added to the oily component, followed by agitation starting at room temperature for 1 hour during ice cooling. The solid product obtained by filtering off the solid precipitate was dried at room temperature for 1 hour under reduced pressure. Thus, 1-O-methyl-5-deoxy-5-chloro-β-D-ribofuranose (65 g; purity: 100%; yield: 53%) was obtained. The sulfur content was not more than 0.3% by weight.

### (Example 2C) Synthesis of 1-O-methyl-5-deoxy-5-chloro-D-ribofuranose

Crude 1-O-methyl-D-ribofuranose (134 g) synthesized as in Example 1 above and acetonitrile (520 mL) and triethylamine (283 g, 2.8 mol) were introduced into a 2-L flask. Thionyl chloride (254 g, 2.1 mol) was slowly added dropwise thereto during ice cooling. Thereafter, the internal temperature was increased from 60°C to 65°C, followed by agitation with heating for 2 hours. The flask was cooled to room temperature. Then, triethylamine hydrochloride that had precipitated from the obtained reaction solution was filtered off, followed by washing with acetonitrile (300 mL). The filtrate was concentrated under reduced pressure and ethyl acetate (700 mL) and 28% ammonia water (365 g) were added thereto so as to cause a reaction at room temperature for I hour. After separating the liquid from the organic layer and the aqueous layer, extraction operation was repeated 3 times with the use of ethyl acetate (200 mL). Activated carbon (manufactured by Wako Pure Chemical Industries, Ltd., 10 g) was added to the organic layer of the resultant, followed by agitation for 30 minutes. Then, activated carbon was filtered off, and then washed by sprinkling ethyl acetate (200 mL). The filtrate was concentrated under reduced pressure. Thus, crude 1-O-methyl-5-deoxy-5-chloro-D-ribofuranose (114 g; purity: 89%; yield: 83%) was obtained as a brown oily component. The sulfur content was not more than 0.3% by weight.

### (Example 3A) Synthesis of 1-O-methyl-5-deoxy-D-ribofuranose

1-O-methyl-5-deoxy-5-chtoro-D-ribofuranose (3.0 g, 16.4 mmol) synthesized as in Example 2, 2-propanol (15 mL), and Na₂CO₃ (2.09 g, 19.7 mmol) were introduced into a 70-mL autoclave containing sponge nickel (manufactured by Nikko Rica Corporation, 3.0 g), followed by a reaction at a hydrogen pressure of 0.5 MPa and an internal temperature of 90°C for 4 hours. The temperature and the pressure were adjusted to ordinary temperature and pressure. Then, NaOH (0.67 g, 16.4 mmol) was added thereto, followed by a reaction at a pressure of 0.5 MPa and an internal temperature of 90°C for 2 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, crude 1-O-methyl-5-deoxy-D-ribofuranose (3.04 g; purity: 67%; yield: 84%) was obtained as a colorless oily component.
[NMR data]
¹H-NMR(400MHz,CDCl₃):δ(β-anomer)1.35(d,J=4.00Hz,3H), 3.38(s,3H), 4.03-4.01(m,3H),4.81(s,1H)
(α-anomer)1.30(d.J=6.32Hz,3H),2.73(d,J=8.63Hz,1H),2.99(d,J=8.32Hz,1H),3.47(s,3H). 3.64-3.60(m,1H),4.03-3.99(m,1H),4.14-4.00(m,1H),4.91(d,H=4.56Hz,1H)

### (Example 3B) Synthesis of 1-O-methyl-5-deoxy-D-ribofuranose

1-O-methyl-5-deoxy-5-chloro-D-ribofufanose (3.0 g, 16.4 mmol), 2-propanol (15 mL), and Na₂CO₃ (2.09 g, 19.7 mmol) were introduced into a 70-mL autoclave containing sponge nickel (manufactured by Nikko Rica Corporation, 3.0 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-O-methyl-5-deoxy-D-ribofuranose (2.34 g; purity: 90%; yield: 87%) was obtained as a colorless oily component.

### (Example 3C) Synthesis of 1-O-methyl-5-deoxy-D-ribofuranose

1-O-methyl-5-deoxy-5-chloro-D-ribofuranose (3.0 g, 16.4 mmol), 2-propanul (15 mL), and Na₂CO₃ (2.09 g, 19.7 mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 4 hours, The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-O-methyl-5-deoxy-D-ribofuranose (2.13 g; purity: 90%; yield: 79%) was obtained as a colorless oily component.

### (Example 3D) Synthesis of 1-O-methyl-5 -deoxy-D-ribofuranose

1-O-methyl-5-deoxy-5-chloro-β-D-ribofuranose (3.0 g, 16.4 mmol), 2-propanol (15 mL), and triethylamine (2.0 g, 19.7mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-O-methyl-5-deoxy-D-ribofuranose (1.05 g; purity: 90%; yield: 39%) was obtained as a colorless oily component.

### (Example 3E) Synthesis of 1-O-methyl-5-deoxy-β-D-ribofuranose

1-O-methyl-5-deoxy-5-chloro-β-D-ribofuranose (3.0 g, 16.4 mmol), 2-propanol (15 mL), and K₂CO₃ (2.7 g, 19.7 mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-O-methyl-5-deoxy-β-D-ribofuranose (2.24 g; purity: 90%; yield: 83%) was obtained as a colorless oily component.

### (Example 3F) Synthesis of 1-O-methyl-5-deoxy-D-ribofuranose

1-O-methyl-5-deoxy-5-chloro-D-ribofuranose (3.0 g, 16.4 mmol), 2-propanol (15 mL), and DBU (3.0 g, 19.7 mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-P-methyl-5-deoxy-D-ribofuranose (1.21 g; purity: 90%; yield: 45%) was obtained as a colorless oily component.

### (Example 3G) Synthesis of 1-O-methyl-5-deoxy-D-ribofuranose

1-O-methyl-5-deoxy-5-chloro-D-ribofuranose (3.0 g, 16.4 mmol), 2-propanol (15 mL), and 23% ammonia water (10.1 g, 19.7 mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-O-methyl-5-deoxy-D-ribofuranose (1.05 g; purity: 90%; yield: 39%) was obtained as a colorless oily component.

### (Example 3H) Synthesis of 1-O-methyl-5-deoxy-D-ribofuranose

1-O-methyl-5-deoxy-5-chloro-D-ribofuranose (3.0 g, 16.4 mmol), 2-butanol (15 mL), and Na₂CO₃ (2.09 g, 19.7 mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 3 hours. The temperature and pressure were adjusted to ordinary temperature and pressure. Then, the resulting solid component was filtered off with the use of a Kiriyama funnel having the bottom portion covered with Celite (6.5 g). The separated Celite was washed with 2-propanol (30 mL). Then filtrate was concentrated under reduced pressure. Thus, 1-O-methyl-5-deoxy-D-ribofuranose. (2.19 g; purity; 90%; yield: 81%) was obtained as a colorless oily component.

### (Example 31) Synthesis of 1-O-methyl-5-deoxy-D-ribofuranose

Herein, t-butanol was used as a solvent instead of 2-butanol and the treatment was performed as described above. As a result, 1-O-methyl-5-deoxy-D-ribofuranose (2.1 g; purity; 90%; yield: 78%) was obtained as a colorless oily component.

### (Example 3J) Synthesis of 1-O-methyl-5-deoxy-D-ribofuranose

Herein, isopropyl acetate was used as a solvent instead of 2-butanol and the treatment was performed as described above. As a result, 1-O-methyl-5-deoxy-D-ribofuranose (1.45 g; purity: 90%; yield: 54%) was obtained as a colorless oily component.

### (Example 3K) Synthesis of 1-O-methyl-5-deoxy-D-ribofuranose

Herein, isobutanol was used as a solvent instead of 2-butanol and the treatment was performed as described above. As a result, 1-O-methyl-5-deoxy-D-ribofuranose (2.17 g; purity: 90%; yield: 80%) was obtained as a colorless oily component.

### (Example 3L) Synthesis of 1-O-methyl-5-deoxy-D-ribofuranose

Herein, t-amylalcohol was used as a solvent instead of 2-butanol and the treatment was performed as described above. As a result, 1-O-methyl-5-deoxy-D-ribofuranose (2.01 g; purity: 90%; yield: 74%) was obtained as a colorless oily component.

### (Example 4A) Synthesis of 1,2.3-tri-O-acetyl-5-deoxy-β-D-ribofuranose

1-O-methyl-5-deoxy-D-ribofuranose (2.9 g; α/β = 25/75; purity: 67%; 13.2 mmol) obtained in Example 3 was introduced into a 50-mL, flask. Acetic anhydride (7.3 g, 70.8 mmol) and acetic acid (2.3 g, 39.0 mmol) were added thereto, followed by a reaction at 85°C for 2 hours. TLC (thin layer chromatography) was performed to confirm the disappearance of starting materials, followed by cooling to room temperature. Pyridine (0.92 g, 11 mmol) was added to the resultant. A solution obtained by diluting concentrated sulfuric acid (2.23 g, 22.7 mmol) with acetic acid (2.84 g) was slowly added dropwise thereto during ice cooling so as to cause a reaction at an internal temperature of 2.5°C for 2 hours. Sodium acetate (3.7 g, 45.4 mmol) was added to the resultant, followed by agitation for 30 minutes and then concentration under reduced pressure. Toluene (100 mL) and a saturated sodium hydrogen carbonate aqueous solution (120 mL) were added thereto for extraction. The separated organic layer was washed twice with desalted water (50 mL), followed by drying with anhydrous sodium sulfate (10 g). Thereafter, a filtrate obtained by filtering off solid components was concentrated under reduced pressure. 1,2,3-tri-O-acetyl-5-deoxy-D-ribofuranose (3.21 g; purity: 89%; yield: 83%) was obtained as a colorless oily component. This oily component was analyzed by liquid chromatography, and α/β = 30/70 was found.

2-propanol (2.8 mL) was added to the obtained oily component. Water (5.4 mL) was added thereto. The crystalline precipitate was filtered off and dried under reduced pressure at room temperature for 1 hour. Thus, 1,2,3-tri-O-acetyl-5-deoxy-β-D-ribofuranose (1.33 g; purity: 100%; yield: 39%) was obtained.
[NMR data]
¹H-NMR(400MHz,CDCl₃):δ(β-anomer)1.37(d,J=6.56Hz,3H),2.07(s,3H),2.09(s,3H),2.12 (s,3H), 4.28(dq,J=6.56,6.36Hz,1H),5.10(dd,J=1.76,6.80Hz,1H), 5.34(dd,J=1.0,4.8Hz,1H),6.12(d,J=1.28Hz,1H)

### (Example 4B) Synthesis of 1,2,3-tri-O-acetyl-5-deoxy-β-D-ribofuranose

1-O-methyl-5-deoxy-D-ribofuranose (2,0 g; α/β = 25/75; 13.5 mmol) was introduced into a 100-mL flask. Sodium acetate (0.22 g, 2.7 mmol), acetic anhydride (4.1 g, 41 mmol), and dibutyl ether (1 mL) were added thereto, followed by a reaction at 85°C for 5 hours and then cooling to room temperature. Pyridine (0.11 g, 1.4 mmol) was added thereto. Concentrated sulfuric acid (0.40 g, 4.1 mmol) was slowly added dropwise to the resultant during ice cooling, followed by agitation at room temperature for 5 hours. Heptane (8 mL) was added thereto, followed by agitation at -20°C for 5 hours. Saturated sodium bicarbonate (20 mL) was added thereto, followed by agitation for 30 minutes. Then, the resultant was extracted twice with the use of toluene (50 mL) such that the organic layer was separated. The separated organic layer was washed twice with desalted water (5 mL). The thus separated organic layer was concentrated under reduced pressure. 1,2,3-tri-O-acetyl-5-deoxy-D-ribofuranose (2.1 g; purity: 90%; yield: 54%) was obtained as a colorless oily component. This oily component was analyzed by liquid chromatography. α/β = 12/88 was found.

2-propanol (2 mL) was added to the oily component, followed by agitation at 0°C for 2 hours. The crystalline precipitate was filtered off and dried under reduced pressure at room temperature for 1 hour. Thus, 1,2,3-tri-O-acetyl-5-deoxy-β-D-ribofuranose (1.3 g; purity: 100%; yield: 38%) was obtained.

### (Example 4C) Synthesis of 1,2,3-tri-O-acetyl-5-deoxy-β-D-ribofuranose

1-O-methyl-5-deoxy-D-ribofuranose (2.0 g; α/β = 25/75; 13.5 mmol) was introduced into a 100-mL flask. Sodium acetate (0.22 g, 2.7 mmol), acetic anhydride (4.1 g, 41 mmol), and toluene (1 mL) were added thereto, followed by a reaction at 85°C for 5 hours and then cooling to room temperature. Pyridine (0.11 g, 1.4 mmol) was added thereto. Concentrated sulfuric acid (0.40 g, 4.1 mmol) was slowly added dropwise to the resultant during ice cooling, followed by agitation at room temperature for 5 hours. Heptane (8 mL) was added thereto, followed by agitation at -20°C for 5 hours. Saturated sodium bicarbonate (20 mL) was added thereto, followed by agitation for 30 minutes. Then, the resultant was extracted twice with the use of toluene (50 mL) such that the organic layer was separated. The separated organic layer was washed twice with desalted water (5 mL). The thus separated organic layer was concentrated under reduced pressure. 1,2,3-tri-O-acetyl-5-deoxy-D-ribofuranose (2.0 g; purity: 80%; yield: 46%) was obtained as a colorless oily component. This oily component was analyzed by liquid chromatograph. α/β = 13/87 was found.

2-propanol (1 mL) was added to the oily component, followed by agitation at 0°C for 2 hours. The crystalline precipitate was filtered off and dried under reduced pressure at room temperature for 1 hour. Thus, 1,2,3-tri-O-acetyl-5-deoxy-β-D-ribofuranose (1.1 g; purity: 100%; yield: 31%) was obtained.

### (Example 4D) Synthesis of 1,2,3-tri-O-acetyl-5-deoxy-β-D-ribofuranose

1-O-methyl-5-deoxy-D-ribofuranose (10 g; α/β = 25/75; 67.5 mmol) was introduced into a 200-mL flask. Sodium acetate (0.55 g, 6.8 mmol), acetic anhydride (27.8 g, 270 mmol), and acetic acid (12.2 g, 203 mmol) were added thereto, followed by a reaction at 85°C for 2 hours and then cooling to room temperature. Then, toluene (50 mL) and saturated sodium bicarbonate (40 mL) were added to the resultant, followed by agitation for 30 minutes. Thereafter, the organic layer was separated, The separated aqueous layer was extracted with toluene (30 mL). The organic layer was added thereto, followed by washing with desalted water (10 mL), followed by concentration under reduced pressure. 1-O-methyl-2;3-di-O-acetyl-5-deoxy-D-ribofuranose (15.6 g; yield: 100%) was obtained as a colorless oily component. The oily component was analyzed by NMR. α/β = 25/75 was found.

### (Example 4E) Synthesis of 1,2,3-tri-O-acetyl-5-deoxy-D-ribofuranose

1-O-methyl-2,3-di-O-acetyl-5-deoxy-D-ribofuranose (1 g; α/β = 25/75, 4.3 mmol) was introduced into a 30-mL flask. Dibutyl ether (1 mL) and acetic anhydride (0.44 g, 4.3 mmol) were added thereto. Sulfuric acid (0.10 g, 1.1 mmol) was added to the resultant during ice cooling. A reaction took place at room temperature for 2 hours, followed by cooling at -20°C. Heptane (3 mL) was added to the resultant, followed by agitation for 3 hours. Saturated sodium bicarbonate (20 mL) was added thereto, followed by agitation for 15 minutes. Extraction was carried out twice with the use of ethyl acetate (50 mL) for reparation of the organic layer. The separated organic layer was washed twice with desalted water (10 mL). The separated organic layer was concentrated under reduced pressure. 1,2,3-tri-O-acetyl-5-deoxy-D-ribofuranose (1.2 g; purity: 47%; yield: 52%) was obtained as a colorless oily component. This oily component was analyzed by liquid chromatography. α/β = 16/84 was found.

### (Example 4F) Synthesis of 1,2,3-tri-O-acetyl-5-deoxy-β-D-ribofuranose

1-O-methyl-2,3-di-O-acetyl-5-deoxy-D-ribofuranose (2 g; α/β = 30/70; 8.6 mol) was introduced into a 100-mL flask. Dibutyl ether (4 mL), acetic anhydride (1.1 g, 10.3 mmol), and pyridine (0.27 g, 3.4 mmol) were added thereto. Sulfuric acid (0.67 g, 6.9 mmol) was added dropwise to the resultant during ice cooling so as to cause a reaction at room temperature for 5 hours. Saturated sodium bicarbonate (20 mL) was added thereto, followed by agitation for 30 minutes. Extraction was carried out twice with the use of dibutyl ether (50 mL) for separation of the organic layer. The separated organic layer was washed twice with desalted water (10 mL). The separated organic layer was concentrated under reduced pressure. 1,2,3-tri-O-acetyl-5-deoxy-D-ribofuranose (2.1 g; purity: 85%; yield: 80%) was obtained as a colorless oily component. This oily component was analyzed by liquid chromatography. α/β = 10/90 was found. 2-propanol (2 mL) was added to the oily component, followed by agitation at 0°C for 2 hours. The crystalline precipitate was filtered off and dried under reduced pressure at room temperature for 1 hour. Thus, 1,2,3-tri-O-acetyl-5-deoxy-β-D-ribofuranose (1.3 g; purity: 100%; yield: 58%) was obtained.

### (Example 4G) Synthesis of 1,2,3-tri-O-acetyl-5-deoxy-β-D-ribofuranose

1-O-methyl-2,3-di-O-acetyl-5-deoxy-D-ribofuranose (2.0 g; α/β = 25/75; 8.6 mmol) was introduced into a 100-mL flask. Heptane (4 mL), acetic anhydride (1.1 g, 10.3 mmol), and pyridine (0.27 g, 3.4 mmol) were added thereto. Sulfuric acid (0.67 g, 6.9 mmol) was added dropwise to the resultant during ice cooling so as to cause a reaction at room temperature for 5 hours, followed by cooling at -20°C. Heptane (3 mL) was added to the resultant, followed by agitation for 3 hours. Saturated sodium bicarbonate (20 mL) was added thereto, followed by agitation for 30 minutes. Extraction was carried out twice with the use of ethyl acetate (50 mL) for separation of the organic layer. The separated organic layer was washed twice with desalted water (10 mL). The separated organic layer was concentrated under reduced pressure. 1,2,3-tri-O-acetyl-5-deoxy-D-ribofuranose (1.8 g; purity: 75%; yield: 60%) was obtained as a colorless oily component. This oily component was analyzed by liquid chromatography, α/β = 10/90 was found. 2-propanol (1 mL) was added to the oily component, followed by agitation at 0°C for 2 hours. The crystalline precipitate was filtered off and dried under reduced pressure at room temperature for 1 hour. Thus, 1,2,3-tri-O-acetyl-5-deoxy-β-D-ribofuranose (1.2 g; purity: 100%; yield: 54%) was obtained.

### (Example 4H) Synthesis of 1,2,3-tri-O-acetyl-5-deoxy-D-riboforanose

1,2,3-tri-O-acetyl-5-deoxy-D-ribofuranose (1.0 g; α/β = 25/75; 3.84 mmol) was introduced into a 10-mL flask, Diisopropyl ether (2 mL) and pyridine (0.06 mL, 0.77 mmol) were added thereto. Sulfuric acid (0.15 g, 1.5 mmol) was added to the resultant during ice cooling so as to cause a reaction at 0°C for 2 hours. Thereafter, saturated sodium bicarbonate was added thereto. Extraction was carried out twice with ethyl acetate (30 mL). 1,2,3-tri-O-acetyl-5-deoxy-D-ribofuranose (0.75 g; purity: 75%; yield: 56%) was obtained as a colorless oily component. This oily component was analyzed by liquid chromatography. α/β = 10/90 was found.

### (Example 41) Synthesis of 1,2,3-tri-O-acetyl-5-deoxy-β-D-ribofuranose

1,2,3-tri-O-acetyl-5-deoxy-D-ribofuranose (1.0 g; α/β = 90/10, 3.84 mmol) was introduced into a 10-mL flask. Diisopropyl ether (2 mL), acetic anhydride (0.18 mL, 1.9 mmol), and pyridine (0.06 mL, 0.77 mmol) were added thereto. Sulfuric acid (0.15 g, 1.5 mmol) was added to the resultant during ice cooling so as to cause a reaction at 0°C for 2 hours. Thereafter, saturated sodium bicarbonate was added thereto. Extraction was carried out twice with ethyl acetate (30 mL). 1,2,3-tri-O-acetyl-5-deoxy-D-ribofuranose (1.1 g; purity: 85%; yield: 95%) was obtained, as a colorless oily component. This oily component was analyzed by liquid chromatography, α/β = 5/95 was found. 2-propanol (3 mL) was added to the obtained oily component, followed by agitation during ice cooling for 2 hours. The crystalline precipitate was filtered off and dried under reduced pressure at room temperature for I hour. Thus, 1,2,3-tri-O-acetyl-5-deoxy-β-D-ribofuranose (0.71 g; purity: 100%; yield: 71%) was obtained.

### (Example 4J) Synthesis of 1,2,3-tri-O-acetyl-5-deoxy-D-ribofuranose

1,2,3-tri-O-acetyl-5-deoxy-D-ribofuranose (1.0 g; α/β = 25/75; 3.84 mmol) was introduced into a 10-mL flask. Toluene (0.09 mL), pyridine (0.05 mL, 0.19 mmol), and heptane (0.9 mL) were added thereto. Sulfuric acid (0.04 g, 0.38 mmol) was added to the resultant during ice cooling so as to cause a reaction at 0°C for 2 hours. Thereafter, saturated sodium bicarbonate was added thereto. Extraction was carried out twice with ethyl acetate (30 mL). 1,2,3-tri-O-acetyl-5-deoxy-D-ribofuranose (1.2 g; purity: 62%; yield: 75%) was obtained, as a colorless oily component. This oily component was analyzed by liquid chromatography. α/β = 9/91 was found.

### (Example 4K) Synthesis of 1,2,3-tri-O-acetyl-5-deoxy-D-ribofuranose

1,2,3-tri-O-acetyl-5-deoxy-D-ribofuranose (1.0 g; α/β = 25/75; 3.84 mmol) was introduced into a 20-mL flask. Diisopropyl ether (2 mL) was added thereto. Sulfuric acid (0.038 g, 0.38 mmol) was added to the resultant during ice cooling so as to cause a reaction at -20°C for 3 hours. Thereafter, saturated sodium bicarbonate (10 mL) was added thereto. Extraction was carried out twice with ethyl acetate (30 mL). 1,2,3-tri-O-acetyl-5-deoxy-D-ribofu-ranose (1.1 g; purity: 47%; yield: 52%) was obtained as a colorless oily component. This oily component was analyzed by liquid chromatography. α/β = 10/90 was found.

### (Example 4L) Synthesis of 1,2,3-tri-O-acetyl-5-deoxy-β-D-ribofuranose

1,2,3-tri-O-acetyl-5-deoxy-D-ribofuranose (1.0 g; α/β = 25/75, 3.84 mmol) was introduced into a 10-mL flask. Diisopropyl ether (2 mL), acetic anhydride (0.18 mL, 1.9 mmol), and pyridine (0.06 mL, 0.77 mmol) were added thereto. Sulfuric acid (0.15 g, 1.5 mmol) was added to the resultant during ice cooling so as to cause a reaction at 0°C for 2 hours. Thereafter, saturated sodium bicarbonate was added thereto. Extraction was carried out twice with ethyl acetate (30 mL). 1,2,3-tri-O-acetyl-5-deoxy-D-ribofuranose (1.2 g; purity: 8 1 %; yield: 95%) was obtained as a colorless oily component. This oily component was analyzed by liquid chromatography. α/β = 8/92 was found. 2-propanol (3 mL) was added to the obtained oily component, followed by agitation during ice cooling for 2 hours. The crystalline precipitate was filtered off and dried under reduced pressure at room temperature for 1 hour. Thus, 1,2,3-tri-O-acetyl-5-deoxy-β-D-ribofuranose (0.71 g; purity: 100%; yield: 71%) was obtained.

### (Example 4M) Synthesis of 1,2,3-tri-O-acetyl-5-deoxy-β-D-ribofuranose

1,2,3-tri-O-acetyl-5-deoxy-D-ribofuranose (1.0 g; α/β = 25/75, 3.84 mmol) was introduced into a 10-mL flask. Dibutyl ether (2 mL), acetic anhydride (0.18 mL, 1.9 mmol), and pyridine (0.06 mL, 0.77 mmol) were added thereto. Sulfuric acid (0.15 g, 1.5 mmol) was added dropwise to the resultant during ice cooling so as to cause a reaction at 0°C for 2 hours. Thereafter, saturated sodium bicarbonate was added thereto. Extraction was carried out twice with ethyl acetate (30 mL). 1,2,3-tri-O-acetyl-5-deoxy-D-ribofuranose (1.4 g; purity: 69%; yield: 97%) was obtained as a colorless oily component. This oily component was analyzed by liquid chromatography. α/β = 4/96 was found. 2-propanol (3 mL) was added to the obtained oily component, followed by agitation during ice cooling for 2 hours. The crystalline precipitate was filtered off and dried under reduced pressure at room temperature for 1 hour. Thus, 1,2,3-tri-O-acetyl-5-deoxy-β-D-ribofuranose (0.74 g; purity: 100%; yield: 74%) was obtained.

### (Example 4N) Synthesis of 1,2,3-tri-O-acetyl-5-deoxy-β-D-ribofuranose

1,2,3-tri-O-acetyl-5-deoxy-D-ribofuranose (1.0 g; α/β = 25/75, 3.84 mmol) was introduced into a 10-mL) flask. Hexane (2 mL), acetic anhydride (0.18 mL, 1.9 mmol), and pyridine (0.06 mL, 0.77 mmol) were added thereto. Sulfuric acid (0.15 g, 1.5 mmol) was added dropwise to the resultant, during ice cooling so as to cause a reaction at 0°C for 2 hours. Thereafter, saturated sodium bicarbonate was added thereto. Extraction was carried out twice with ethyl acetate (30 mL). 1,2,3-tri-O-acetyl-5-deoxy-D-ribofuranose (1.6 g; purity: 60%; yield: 95%) was obtained as a colorless oily component. This oily component was analyzed by liquid chromatography. α/β = 4/96 was found. 2-propanol (3 mL) was added to the obtained oily component, followed by agitation during ice cooling for 2 hours, The crystalline precipitate was filtered off and dried under reduced pressure at room temperature for 1 hour. Thus, 1,2,3-tri-O-acetyl-5-deoxy-β-D-ribofuranose (0.74 g; purity: 100%; yield: 74%) was obtained.

### (Example 40) Synthesis of 1,2,3-tri-O-acetyl-5-deoxy-β-D-ribofuranose

1,2,3-tri-O-acetyl-5-deoxy-D-ribofuranose (20 g; α/β = 25/75, 76.9 mmol) was introduced into a 500-mL) flask. Toluene (25 mL), acetic anhydride (1.4 mL, 15.4 mmol), and pyridine (0.31 mL, 3.85 mmol) were added thereto, Heptane (25 mL) was added to the resultant. Sulfuric acid (0.75 g, 7.69 mmol) was added dropwise thereto during ice cooling. Further, heptane (50 mL) was added thereto so as to cause a reaction during ice cooling for 2 hours. Thereafter, saturated sodium bicarbonate (40 mL) was added thereto. Extraction was carried out twice with toluene (220 mL). The resultant was washed with desalted water (20 mL). The separated organic layer was concentrated under reduced pressure. Thus, 1,2,3-tri-O-acetyl-5-deoxy-D-ribofuranose (22.1 g; purity: 86%; yield: 95%) was obtained as a colorless oily component, This oily component was analyzed by liquid chromatography. α/β = 3/97 was found. 2-propanol (67 mL) was added to the obtained oily component, followed by agitation during ice cooling for 2 hours. The crystalline precipitate was filtered off and dried under reduced pressure at room temperature for I hour. Thus, 1,2,3-tri-O-acetyl-5-deoxy-β-D-ribofuranose (14.1 g; purity: 100%; yield: 70%) was obtained.

### (Example 4P) Synthesis of 1,2,3-tri-O-acetyl-5-deoxy-β-D-ribofuranose

1,2,3-tri-O-acetyl-5-deoxy-D-ribofuranose (20 g; α/β = 25/75, 76.9 mmol) was introduced into a 500-mL flask. Toluene (10 mL), acetic anhydride (1.4 mL, 15.4 mmol), and pyridine (0.31 mL, 3.85 mmol) were added thereto. Heptane (10 mL) was added to the resultant. Sulfuric acid (0.75 g, 7.69 mmol) was added dropwise thereto during ice cooling. Further, heptane (40 mL) was added thereto so as to cause a reaction during ice cooling for 2 hours. Thereafter, saturated sodium bicarbonate (40 mL) was added thereto. Extraction was carried out twice with toluene (100 mL). The resultant was washed with desalted water (10 mL). The separated organic layer was concentrated under reduced pressure. Thus, 1,2,3-trz-O-acetyl-5-deoxy-D-ribofuranose (21.5 g; purity: 89%; yield: 96%) was obtained as a colorless oily component. This oily component was analyzed by liquid chromatography. α/β = 4/96 was found. 2-propanol (40 mL) was added to the obtained oily component, followed by agitation during ice cooling for 2 hours. The crystalline precipitate was filtered off and dried under reduced pressure at room temperature for 1 hour. Thus, 1,2,3-tri-O-acetyl-5-deoxy-β-D-ribofuranose (15.2 g; purity: 100%; yield: 76%) was obtained.

### (Example 5A) Synthesis of 1,2,3-tri-O-acetyl-5-deoxy-5-chloro-β-D-ribofuranose

1-O-methyl-5-deoxy-5-chloro-D-ribofuranose (2.0 g; α/β = 30/70; 11.0 mmol) was introduced into a 20-mL flask. Sodium acetate (0.18 g, 2.2 mmol), acetic anhydride (4.5 g, 44.0 mmol), and dibutyl ether (4 mL) were added thereto so as to cause a reaction at 85°C for 5 hours. The resultant was cooled to room temperature. Pyridine (0.09 g, 1.1 mmol) was added thereto. Concentrated sulfuric acid (0.54 g, 5.5 mmol) was slowly added dropwise thereto during ice cooling so as to cause a reaction at room temperature for 5 hours, followed by cooling to -20°C. Heptane (10 mL) was added thereto, followed by agitation for 3 hours. Saturated sodium bicarbonate (20 mL) was added to the resultant, followed by agitation for 30 minutes, Then, extraction was carried out twice with the use of ethyl acetate (50 mL) for separation of the organic layer. The separated organic layer was washed twice with desalted water (10 mL). Then, the separated organic layer was concentrated under reduced pressure. 1,2,3-tri-O-acetyl-5-deoxy-5-chloro-D-ribofuranose (3.0 g; purity: 80%; yield: 75%) was obtained as an oily component. This oily component was analyzed by liquid chromatography (α/β = 8/92). 2-Propanol (2 mL) was added to the oily component, followed by agitation during ice cooling for 2 hours. The crystalline precipitate was filtered off and dried under reduced pressure at room temperature for 1 hour. Thus, 1,2,3-tri-O-acetyl-5-deoxy-5-chloro-β-D-ribofuranose (1.8 g; purity: 100%; yield: 56%) was obtained.

### (Example 5B)

### Synthesis of 1-O-methyl-2,3-di-O-acetyl-5-deoxy-5-chloro-D-ribofuranose

1-O-methyl-5-deoxy-5-chloro-D-ribofuranose (50 g; α/β = 30/70; 274 mmol) was introduced into a 500-mL flask. Sodium acetate (11.2 g, 137 mmol), acetic anhydride (70 g, 685 mmol), and acetic acid (60 g, 1000 mmol) were added thereto, followed by a reaction at 85°C for 2 hours. The resultant was cooled to room temperature. Toluene (250 mL) was added to the reaction solution. The reaction solution was added to desalted water (620 mL), followed by agitation for 30 minutes for separation of the organic layer. The separated organic layer was washed three times with saturated sodium bicarbonate (540 mL). The resultant was washed with desalted water (180 mL). The organic layer was concentrated under reduced pressure. 1-O-methyl-2,3-di-O-acetyl-5-deoxy-5-chloro-D-ribofurauose (53.1 g; purity: 93%; yield: 67%) was obtained as a colorless oily component, This oily component was analyzed by NMR (α/β = 30/70). As a result of analysis of ¹H NMR spectral data, the component was identified as 1-O-methyl-2,3-di-O-acetyl-5-deoxy-5-chloro-D-ribofuranose. [NMR data]
¹H-NMR(400MHz,CDC13):δ(β-anomer)2.07(s,3H),2.12(s,3H),3.40(s,3H),3.64(dd,J=6.08 , 11.4Hz,1H),3.70(dd,J=5.32;11.4Hz;1H),4.32(m,1H),4.92(s,1H),5.25(d,J=5.08Hz,1H),5. 35(dd,J=1.52,5.04Hz,1H)
(α-anomer)2.02(s,3H),2.14(s,3H),3.46(s,3H),3.78(dd,J=3.56,11.9Hz,1H),3.86(dd,J=3.76, 11.9Hz, 1H),4.35(m,1H),5.00(m,1H),5.17-5.21(m, 2H)

### (Example 5C) Synthesis of 1,2,3-tri-O-acetyl-5-deoxy-5-chloro-β-D-ribofuranose

1-O-methyl-2,3-di-O-acetyl-5-deoxy-5-chloro-D-ribofuranose (2.0 g; α/β = 30/70; 7.5 mmol) was introduced into a 50-mL flask. Diisopropyl ether (6 mL), acetic anhydride (1.5 g, 15 mmol), and pyridine (0.87 g, 6 mmol) were added thereto. Sulfuric acid (1.6 g, 16.5 mmol) was added dropwise to the resultant during ice cooling so as to cause a reaction during ice cooling for 2 hours. Then, saturated sodium bicarbonate (20 mL) was added thereto, followed by agitation for 30 minutes. Extraction was carried out with the use of diisopropyl ether (20 mL) for separation af the organic layer, The separated organic layer was washed with desalted water (10 mL). The separated organic layer was concentrated under reduced pressure. 1,2,3-tri-O-acetyl-5-deoxy-5-chloro-D-ribofuranose (1.5 g; purity: 87%; yield: 61%) was obtained as a colorless oily component. This oily component was analyzed by liquid chromatography (α/β = 7/93). 2-Propanal (3 mL) was added to the oily component, followed by agitation at 0°C for 2 hours. The crystalline precipitate was filtered off and dried under reduced pressure at room temperature for 1 hour. Thus, 1,2,3-tri-acetyl-5-deoxy-5-chloro-β-D-ribofuranose (1.0 g; purity: 100%; yield: 45%) was obtained.
[NMR data]
¹H-NMR(400MHz,CDCl₃):δ2.08(s,3H),2.11(s,3H),
2.14(s,3H),3.65-3.75(m,2H),4.42(m,1H),5.36(d,J=4.8Hz,1H),5.45(dd,J=2.5,7.3Hz,1H),6. 14(s,1H)

### (Example 6A) Synthesis of 1,2,3-tri-O-acetyl-5-deoxy-β-D-ribofuranose

1,2,3-tri-O-acetyl-5-deoxy-5-chloro-β-D-ribofuranose (3.0 g, 10.2 mmol), 2-propanol (30 mL), Na₂CO₃ (1.30 g, 12.2 mmol) were introduced into a 70-mL autoclave containing sponge nickel (manufactured by Nikko Rica Corporation, 1.0 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (4.5 g). The separated Celite was washed with 2-propanol (21 mL). Then, filtrate was concentrated under reduced pressure. Thus, 1,2,3-tri-O-acetyl-5-deoxy-β-D-ribofuranose (2.76 g; purity: 68%; yield: 71%) was obtained as a colorless oily component.

### (Example 6B) Synthesis of 1,2,3-tri-O-acetyl-5-deoxy-β-D-ribofuranose

1;2,3-tri-O-acetyl-5-deaxy-5-chloro-β-D-ribofuranose (3.0 g, 10.2 mmol), 2-propanol (30 mL), Na₂CO₃ (1.30 g, 12.2 mmol) were introduced into a 70-mL autoclave containing sponge nickel (manufactured by Nikko Rica Corporation, 1.5 g), followed by a reaction at a hydrogen pressure of 0.5 MPa aud an internal temperature of 90°C for 4 hours. The temperature and pressure were adjusted to ordinary temperature and pressure. Then, the resulting solid component was filtered off with the use of a Kiriyama funnel having the bottom portion covered with Celite (4.5 g). The separated Celite was washed with 2-propanol (21 mL). Then, filtrate was concentrated under reduced pressure. Thus, 1,2,3-tri-O-acetyl-5-deoxy-β-D-ribofuranose (2.77 g; purity: 70%; yield: 73%) was obtained as a colorless oily component.

### (Example 6C) Synthesis of 1,2,3-tri-O-acetyl-5-deoxy-β-D-ribofuranose

1,2,3-tri-O-acetyl-5-deoxy-5-chloro-β-D-ribofuranose (3.0 g, 10.2 mmol), 2-propanol (30 mL), and Na₂CO₃ (1.30 g, 12.2 mmol) were introduced into a 70-mL autoclave containing sponge nickel (manufactured by Nikko Rica Corporation, 3.0 g), followed by a reaction at a hydrogen pressure of 0.5 MPa and an internal temperature of 90°C for 14 hours. The temperature and pressure were adjusted to ordinary temperature and pressure. Then, the resulting solid component was filtered off with the use of a Kiriyama funnel having the bottom portion covered with Celite (4.5 g). The separated Celite was washed with 2-propanol (21 mL). Then, filtrate was concentrated under reduced pressure. Thus, 1,2,3-tri-O-acetyl-5-deaxy-β-D-ribofuranose (2.62 g; purity: 70%; yield: 69%) was obtained as a colorless oily component.

### (Example 6D) Synthesis of 1,2,3-tri-O-acetyl-5-deoxy-β-D-ribofuranose

1,2,3-tri-O-acetyl-5-deoxy-5-chloro-β-D-ribofuranose (3.0 g, 10.2 mmol), 2-propanol (12 mL), toluene (3.0 mL), and Na₂CO₃ (1.30 g, 12.2 mmol) were introduced into a 70-mL autoclave containing sponge nickel (manufactured by Nikko Rica Corporation, 1.5 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (4.5 g). The separated Celite was washed with 2-propanol (21 mL) and dichloromethane (12 mL). Then, filtrate was concentrated under reduced pressure. Thus, 1,2,3-tri-O-acetyl-5-deoxy-β-D-ribofuranose (2.65 g; purity: 63%; yield: 63%) was obtained as a colorless oily component.

### (Example 6E) Synthesis of 1,2,3-tri-O-acetyl-5-deoxy-β-D-ribofuranose

1,2,3-tri-O-acetyl-5-deoxy-5-chloro-β-D-ribofuranose (3.0 g, 10.2 mmol), 2-propanol (12 mL), and tetrahydrofuran (3.0 mL), and Na₂CO₃ (1.30 g, 12.2 mmol) were introduced into a 70-mL autoclave containing sponge nickel (manufactured by Nikko Rica Corporation, 1.5 g), followed by a reaction at a hydrogen pressure of 0.5 MPa and an internal temperature of 90°C for 4 hours. The temperature and pressure were adjusted to ordinary temperature and pressure. Then, the resulting solid component was filtered off with the use of a Kiriyama funnel having the bottom portion covered with Celite (4.5 g). The separated Celite was washed with 2-propanol (21 mL) and dichloromethane (12 mL). Then, filtrate was concentrated under reduced pressure. Thus, 1,2,3-tri-O-acetyl-5-deoxy-β**-**D-ribofuranose (2.58 g; purity: 70%; yield: 68%) was obtained as a colorless oily component.

## Claims

1. A process for producing a compound represented by the formula (3): 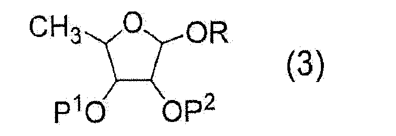 wherein P¹ and P² independently represent a hydrogen atom or an acyl group, OP¹ and Op² may together form an acetal group, and R represents a hydrogen atom, an alkyl group, an aryl group, an aralkyl group, or an acyl group;
which comprises hydrogenating a compound represented by the formula (1) or the formula (2) in the presence of a metal catalyst: 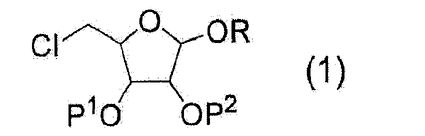 wherein P¹ and P² independently represent a hydrogen atom or an acyl group, OP¹ and OP² may together form an acetal group, and R represents a hydrogen atom, an alkyl group, an aryl groupe, an aralkyl group, or an acyl group; 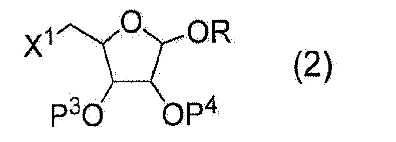 wherein X¹ represents Br or I, P³ and P⁴ independently represent a hydrogen atom or an acyl group, and R represents a hydrogen atom, an alkyl group, an aryl group, an aralkyl group, or an acyl group.

2. The process according to claim 1, wherein P¹ and P² independently represent a hydrogen atom or an acyl group and P³ and P⁴ independently represent a hydrogen atom or an acyl group in the formula (1) or (2).

3. The process according to claim 1 or 2, wherein a hydrogen molecule is allowed to act in the presence of the metal catalyst for hydrogenation.

4. A process for producing a compound represented by the formula (3): 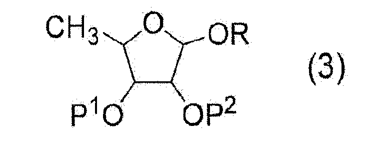 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
which comprises the following steps of:
(a) reacting a compound represented by the formula (4): 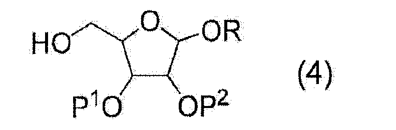 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
with an acid halide or a halogen salt of an acid halide and an alkali metal, and treating the resultant with an acid or an alkali so as to produce a compound represented by the formula (5); 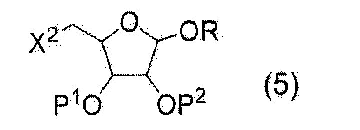 wherein X² represents Cl, Br, or I, P¹ and P² independently represent a hydrogen atom or an acyl group, OP¹ and OP² may together form an acetal group, and R represents a hydrogen atom, an alkyl group, an aryl group, an aralkyl group, or an acyl group; and
(b) hydrogenating the compound represented by the formula (5): 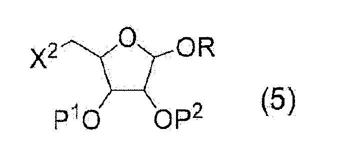 wherein X² represents Cl, Br, or I, P¹ and P² independently represent a hydrogen atom or an acyl group, OP¹ and OP² may together form an acetal group, and R represents a hydrogen atom, an alkyl group, an aryl group, an aralkyl group, or an acyl group;
in the presence of a metal catalyst.

5. A process for producing a compound represented by the formula (6); 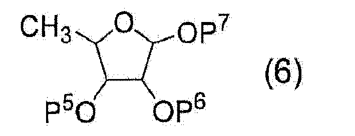 wherein P⁵, P⁶, and P¹ independently represent an acyl group and may be the same or different;
which comprises the following steps of:
(a) producing a compound represented by the formula (3); 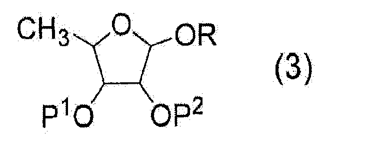 wherein P¹ and P² independently represent a hydrogen atom or an acyl group, OP¹ and OP² may together form an acetal group, and R represents a hydrogen atom, an alkyl group, an aryl group, an aralkyl group, or an acyl group, provided that P¹, P², and R do not simultaneously represent an acyl group;
by the process according to any one of claims 1 to 4; and
(b) converting a hydroxyl group or substituted hydroxyl group in the compound represented by the formula (3) into a hydroxyl group substituted with an acyl group.

6. A process for producing a compound represented by the formula (8); 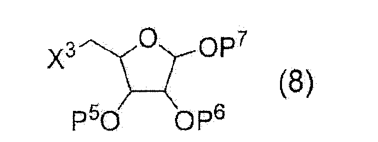
wherein X³ represents Cl, Br, or I and P⁵, P⁶, and P⁷ independently represent an acyl group and may be the same or different;
which comprises the following steps of:
(a) reacting a compound represented by the formula (4): 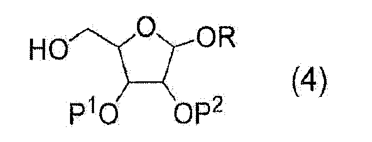 wherein P¹ and P² independently represent a hydrogen atom or an acyl group, OP¹ and OP² may together form an acetal group, and R represents a hydrogen atom, an alkyl group, an aryl group, an aralkyl group, or an acyl group;
with an acid halide or a halogen salt of an acid halide of an alkali metal, and treating the resultant with an acid or an alkali so as to produce a compound represented by the formula (7); 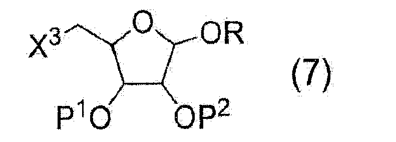 wherein X³ represents Cl, Br, or I, P¹ and P² independently represent a hydrogen atom or an acyl group, OP¹ and OP² may together form an acetal group, and R represents a hydrogen atom, an alkyl group, an aryl group, an aralkyl group, or an acyl group, provided that P¹, P², and R do not simultaneously represent an acyl group; and
(b) converting a hydroxyl group or substituted hydroxyl group of the compound represented by the formula (7) into a hydroxyl group substituted with an acyl group.

7. A process for producing a nucleic acid derivative of the formula (9); 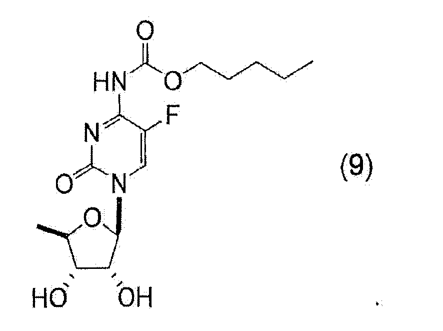 which comprises the following steps of:
(a) producing a compound represented by the formula (6): 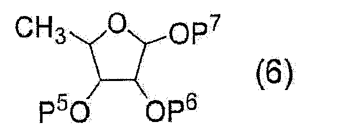 wherein P⁵, P⁶, and P⁷ independently represent an acyl group and may be the same or different;
by the process according to claim 5, and
(b) condensing the compound represented by said formula (6) obtained in the step (a) with 5-fluorocytosines,

8. A process for producing a mixture containing an α-anomer and a β-anomer of a compound of the formula (10); 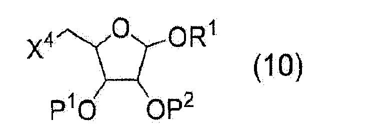 wherein X⁴ represents Cl, Br, I, or a hydrogen atom, P¹ and P² independently represent a hydrogen atom or an acyl group, OP¹ and OP² may together form an acetal group, and R¹ represents an acyl group,
which comprises treating a mixture containing a compound of said formula (10) with an α-configuration at 1-position (α-anomer) and a compound of said formula (10) with a β-configuration at 1-position (β-anomer) in the presence of an acid and a poor solvent, wherein the proportion of the β-anomer in the mixture after treatment becomes greater than that in the mixture before treatment.

9. The process according to claim 8, wherein a base is further allowed to exist during the treatment in the presence of the acid and the poor solvent.

10. The process according to claim 8 or 9, wherein a dehydration agent is Further allowed to exist during the treatment in the presence of the acid and the poor solvent.

11. A process for producing a β-anomer of a compound of the formula (10): 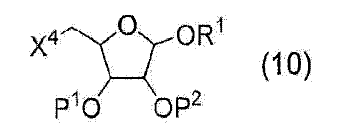 wherein X⁴ represents Cl, Br, I, or a hydrogen atom, P¹ and P² independently represent a hydrogen atom or an acyl group, OP¹ and OP² may together form an acetal group, and R¹ represents an acyl group,
which comprising the following steps of:
(a) producing a mixture containing the compound of said formula (10) with an α -configuration at 1-position (α-anomer) and the compound of said formula (10) with a β-configuration at position (β-anomer) by the process according to any one of claims 8 to 10; and
(b) isolating the β-anomer of a compound of said formula (10) by further purifying the mixture containing an α-anomer and a β-anomer of a compound of said formula (10) produced in the step (a).

12. A process for producing a compound of the formula (10): 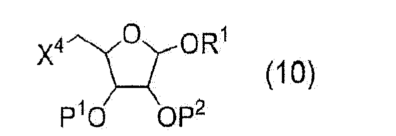 wherein X⁴ represents Cl, Br, I, or a hydrogen atom, P¹ and P² independently represent a hydrogen atom or an acyl group, DP¹ and OP² may together form an acetal group, and R¹ represents an acyl group;
which comprises a step of allowing an acylation agent to act on a mixture containing a compound of the formula (11): 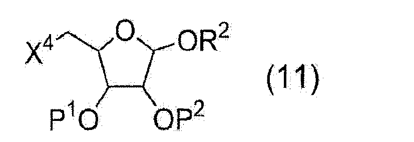 wherein X⁴ represents Cl, Br, I, or a hydrogen atom, P¹ and P² independently represent a hydrogen atom or an acyl group, OP¹ and OP² may together form an acetal group, and R² represents an alkyl group, an aryl group, or an aralkyl group;
with an α-configuration at 1-position (α-anomer) and a compound of said formula (11) with a β-configuration at 1-position (β-anomer) in the presence of an acid and a poor solvent before reaction, so as to obtain a mixture containing the compound of said formula (10) with an α-configuration at 1-position (α-anomer) and the compound of said formula (10) with a β-configuration at 1-position (β-anomer) after reaction, wherein the proportion of the β-anomer in the mixture after reaction becomes greater than that in the mixture before reaction:

13. The process according to claim 12, wherein a base is further allowed to exist when the acylation agent is allowed to act in the presence of the acid and the poor solvent.

14. A process for producing a β-anomer of a compound of the formula (10): 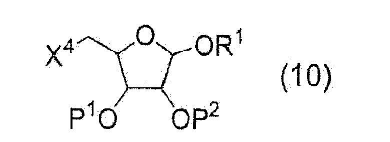 wherein X⁴ represents Cl, Br, I, or a hydrogen atom, P¹ and P² independently represent a hydrogen atom or an acyl group, OP¹ and OP² may together form an acetal group, and R¹ represents an acyl group;
which comprises the following steps of:
(a) producing a mixture containing the compound of said formula (10) with an α -configuration at 1-position (α-anomer) and the compound of said formula (10) with a β-configuration at position (β-anomer) by the process of claim 12 or 13; and
(b) isolating the β-anomer of a compound of said formula (10) by further purifying the mixture containing an α-anomer and a β-anomer of a compound of said formula (10)
produced in the step (a).

15. A D- or L-ribofuranose derivative represented by the formula (12), 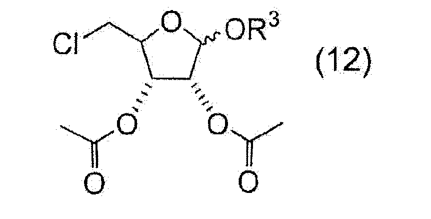 wherein a configuration of 1-positian is α or β and R³ represents an alkyl group with a carbon number of 1 to 6, an aryl group with a carbon number of 6 to 20, or an aralkyl group with a carbon number of 7 to 12.
